(19) Europäisches Patentamt
European Patent Office
Office européen des brevets

(11) EP 3 233 791 B1

(12) EUROPÄISCHE PATENTSCHRIFT

(45) Veröffentlichungstag und Bekanntmachung des Hinweises auf die Patenterteilung:
**12.08.2020 Patentblatt 2020/33**

(21) Anmeldenummer: **15808579.5**

(22) Anmeldetag: **11.12.2015**

(51) Int Cl.:
***C07C 271/28*** *(2006.01)* ***C07C 271/30*** *(2006.01)*
***C07C 321/30*** *(2006.01)* ***G03F 7/00*** *(2006.01)*
***G03F 7/027*** *(2006.01)* ***G03F 7/035*** *(2006.01)*

(86) Internationale Anmeldenummer:
**PCT/EP2015/079385**

(87) Internationale Veröffentlichungsnummer:
**WO 2016/096641 (23.06.2016 Gazette 2016/25)**

(54) **FEUCHTIGKEITSSTABILE HOLOGRAPHISCHE MEDIEN**

MOISTURE STABLE HOLOGRAPHIC MEDIA

MILIEUX HOLOGRAPHIQUES RÉSISTANTS À L'HUMIDITÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.12.2014 EP 14199164**

(43) Veröffentlichungstag der Anmeldung:
**25.10.2017 Patentblatt 2017/43**

(73) Patentinhaber: **Covestro Deutschland AG**
**51373 Leverkusen (DE)**

(72) Erfinder:
- **FÄCKE, Thomas**
  **51375 Leverkusen (DE)**
- **BRUDER, Friedrich-Karl**
  **47802 Krefeld (DE)**
- **RÖLLE, Thomas**
  **51381 Leverkusen (DE)**
- **HÖNEL, Dennis**
  **53909 Zülpich-Wichterich (DE)**

(74) Vertreter: **Levpat**
**c/o Covestro AG**
**Gebäude 4825**
**51365 Leverkusen (DE)**

(56) Entgegenhaltungen:
**EP-A1- 2 154 128 EP-A1- 2 450 387**

- **DATABASE CA [Online] CHEMICAL ABSTRACTS SERVICE, COLUMBUS, OHIO, US; NIE, JUN ET AL: "Preparation method of polyurethane acrylate with high wear and heat resistance", XP002739916, gefunden im STN Database accession no. 2015:317380 & CN 104 371 088 A (CHANGZHOU INSTITUTE OF ADVANCED MATERIALS, BEIJING UNIVERSITY OF CHEMI) 25. Februar 2015 (2015-02-25)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 233 791 B1

**Beschreibung**

Die Erfindung betrifftneue Verbindungen, die sich insbesondere zum Einsatz als Schreibmonomere in holographischen Medien eignen. Weitere Gegenstände der Erfindung sind ein Photopolymer und ein holographischen Medium umfassend die erfindungsgemäßen Verbindungen, sowie eine optische Anzeige, ein Sicherheitsdokument und ein holographisch optisches Element umfassend ein erfindungsgemäßes holographisches Medium.

Für die Verwendungen von Photopolymeren für die Hertsellung holographischer Medienspielt der durch die holographische Belichtung im Photopolymer erzeugte Brechungsindexkontrast $\Delta$n die entscheidende Rolle. Bei der holographischen Belichtung wird das Interferenzfeld aus Signal- und Referenzlichtstrahl (im einfachsten Fall das zweier ebener Wellen) durch die lokale Photopolymerisation von z.B. hochbrechenden Acrylaten an Orten hoher Intensität im Interferenzfeld in ein Brechungsindexgitter abgebildet. Das Brechungsindexgitter im Photopolymeren (das Hologramm) enthält alle Information des Signallichtstrahls. Durch Beleuchtung des Hologramms nur mit dem Referenzlichtstrahl kann dann das Signal wieder rekonstruiert werden. Die Stärke des so rekonstruierten Signals im Verhältnis zur Stärke des eingestrahlten Referenzlichts wird Beugungseffizienz, im folgenden DE wie Diffraction Efficiency, genannt.

Hochbrechende Acrylate sind in der Lage, Brechungsindexgitter mit hoher Amplitude zwischen Bereichen mit niedrigem Brechungsindex und Bereichen mit hohem Brechungsindex zu erzeugen und damit im Photopolymer- Hologramme mit hohem DE und hohem $\Delta$n zu ermöglichen. Dabei ist zu beachten, dass die Gitterstärke und damit das DE vom Produkt aus $\Delta$n und der Photopolymerschichtdicke d abhängt. Die Breite des Winkelbereiches bei dem das Hologramm z.B. bei monochromatischer Beleuchtung sichtbar (rekonstruiert) wird, hängt dann nur noch von der Schichtdicke d ab. Bei Beleuchtung des Hologramms mit z.B. weißem Licht hängt unter einem gegebenen Beleuchtungswinkel die Breite des spektralen Bereiches, der zur Rekonstruktion des Hologramms beitragen kann, ebenfalls nur von der Schichtdicke d ab. Dabei gilt je kleiner d desto größer die jeweiligen Akzeptanzbreiten. Will man daher helle und leicht sichtbare Hologramme herstellen, ist ein hohes $\Delta$n und eine geringe Dicke d anzustreben und zwar so, dass DE möglichst groß wird. Das heißt je höher $\Delta$n wird, desto mehr Freiraum zur Gestaltung der Schichtdicke d für helle Hologramme erreicht man ohne Verlust an DE. Daher kommt der Optimierung von $\Delta$n bei der Optimierung von Photopolymeren eine herausragenden Bedeutung zu (P. Hariharan, Optical Holography, 2nd Edition, Cambridge University Press, 1996).

Aus der WO 2010/0036013 sind Schreibmonomere auf Basis von (substituierten) Phenylcarbamoyloxyethylpropenonaten sowie deren Verwendung als Schreibmonomere in Pho topolymeren zur Herstellung holographischer Medien bekannt. In diese Medien können Hologramme mit hohen Beugungseffzienzen (DE, diffraction efficiency) geschrieben werden.

CN104371088 A offenbart ein Herstellungsverfahren für Polyurethanacrylate mit hoher Trage- und Hitzebeständigkeit.

EP2154128 A offenbart Phenylisocyanat-basierte Urethanacrylate mit hohem Brechungsindex.

Allerdings weisen die bekannten holographischen Medien nicht für alle Anwendungen eine ausreichend hohe Stabilität gegenüber wechselnden Feuchtigkeitsbedingungen auf. So ändert sich die optische Funktion belichteter Medien in Abhängigkeit von der jeweils herrschenden Feuchtigkeit teils erheblich. Dies führt dazu, dass die holographischen Medien nur in einem eng definierten Feuchtigkeitsbereich zuverlässig ihre optische Funktion erfüllen. Umgekehrt verlieren sie ihre Funktion ganz oder zumindest zum Teil, wenn sie in einer Umgebung eingesetzt werden, in der das Feuchtigkeitsniveau außerhalb des engen spezifizierten Bereichs liegt.

Aufgabe der vorliegenden Erfindung war es daher, Verbindungen für die Herstellung holographischer Medien bereit zu stellen, deren Verwendung einerseits das Schreiben von Hologrammen mit einem hohen Brechungsindexkontrast ($\Delta$n) von mehr als 0.025 ermöglicht und andererseits den Einfluss wechselnder Feuchtigkeitsbedingungen auf die Rekonstruktionswellenlänge erheblich reduziert. Insbesondere sollten die Medien bei wechselnden Umgebungsfeuchten eine maximale Veränderung der Rekonstruktionswellenlänge von weniger als 5 nm bezogen auf ein Reflektionshologramm, das durch Interferenz zweier ebener Wellen mit einer Wellenlänge von 532 nm geschrieben wurde, aufweisen.

Diese Aufgabe ist durch ein Photopolymer umfassend Matrixpolymere, Schreibmonomere, monomere Fluorurethane und Photoinitiatoren, wobei die Schreibmonomere Verbindung der Formel (I)

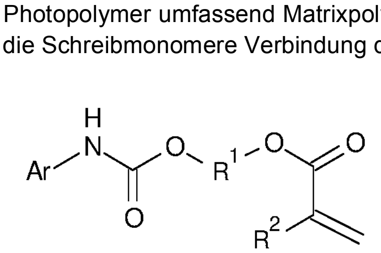

(I);

in der

$R^1$ ein aliphatischer Kohlenwasserstoffrest mit 1-8 C-Atomen ist;

$R^2$ Wasserstoff oder Methyl ist;

Ar ein aromatischer Rest der Formel (II) ist

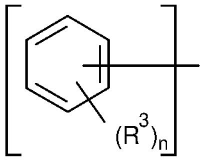

(II),

in der

$R^3$ unabhängig voneinander Reste ausgewählt aus der Gruppe substituiertes oder unsubstituiertes Phenyl, substituiertes oder unsubstituiertes Phenylthiyl, verzweigtes oder unverzweigtes Alkyl, verzweigtes oder unverzweigtes Alkylthiyl, Halogen sind, wobei wenigstens einer der Reste $R^3$ ein Rest ausgewählt aus der Gruppe substituiertes oder unsubstituiertes Phenyl, substituiertes oder unsubstituiertes Phenylthiyl ist;

n = 1 bis 5 ist;

oder Ar ein aromatischer Rest der Formel (III) ist

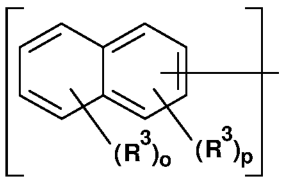

(III),

in der

$R^3$ unabhängig voneinander Reste ausgewählt aus der Gruppe substituiertes oder unsubstituiertes Phenyl, substituiertes oder unsubstituiertes Phenylthiyl, verzweigtes oder unverzweigtes Alkyl, verzweigtes oder unverzweigtes Alkylthiyl, Halogen sind, wobei wenigstens einer der Reste $R^3$ ein Rest ausgewählt aus der Gruppe substituiertes oder unsubstituiertes Phenyl, substituiertes oder unsubstituiertes Phenylthiyl ist.

o = 1 bis 3;

p = 1 bis 4 ist,

wobei die Verbindung der Formel (I) nur eine strahlenhärtende Gruppe aufweist, umfassen, gelöst.

Weiterhin wird die Aufgabe durch die Verbindung der Formel (I')

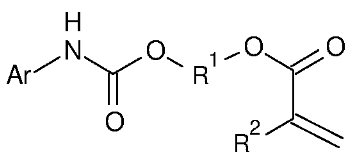

(I');

in der

$R^1$ ein aliphatischer Kohlenwasserstoffrest mit 1-8 C-Atomen ist;

$R^2$ Wasserstoff oder Methyl ist;

Ar ein aromatischer Rest der Formel (II') ist

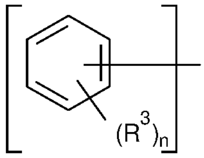

(II'),

in der

$R^{3'}$ unabhängig voneinander Reste ausgewählt aus der Gruppe unsubstituiertes Phenyl, substituiertes oder unsubstituiertes Phenylthiyl, verzweigtes oder unverzweigtes Alkyl, verzweigtes oder unverzweigtes Alkylthiyl, Halogen sind, wobei wenigstens einer der Reste $R^{3'}$ ein Rest ausgewählt aus der Gruppe unsubstituiertes Phenyl, substituiertes oder unsubstituiertes Phenylthiyl ist;

n = 1 bis 5 ist;

oder Ar ein aromatischer Rest der Formel (III) ist

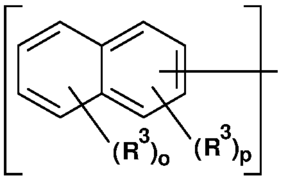

(III),

in der

$R^3$ ein mit einem oder mehreren Phenyl-, Phenythiyl-, Alkyl-, Alkylthiyl-, Halogen, Biphenyl, Naphtyl-Resten substituierter Phenyl- oder Phenylthiyl-Rest ist.

o = 1 bis 3;

p = 1 bis 4 ist,

wobei die Verbindung der Formel (I') nur eine strahlenhärtende Gruppe aufweist, gelöst.

So wurde überraschenderweise gefunden, dass mit Hilfe der erfindungsgemäßen Verbindungen holographische Medien erhalten werden können, die deren Funktion im belichteten Zustand besonders auch bei wechselnden Feuchtigkeitsbedingungen nicht oder nur im geringen Maße beeinflust wird. Diese Medien weisen darüber hinaus einen einen hohen Brechungsindexkontrast ($\Delta n$) auf.

Erfindungsgemäß werden als strahlenhärtende Gruppen solche funktionellen Gruppen bezeichnet, die bei Anwesenheit von Starterradikale, die durch Einwirkung von aktinischer Strahlung erzeugt werden, radikalisch polymersiert werden können. Beispiele für strahlenhärtende Gruppen sind die Acrylat- und die Methacrylat-Gruppe.

Die erfindungsgemäßen Verbindungen können beispielsweise durch Urethanisierung eines entsprechend substituierten Phenylisocyanats mit einem Hydroxyalkylacrylat hergestellt werden. Die Urethanisierung kann bei 60-120 °C unter Einsatz eines Urethanisierungskatalysators durchgeführt werden.

Geeignete Katalysatoren sind tertiäre Amine, Zinn-, Zink-, Eisen- oder Bismuthverbindungen, insbesondere Triethylamin, 1,4-Diazabicycl-[2,2,2]-octan, Bismuthoktoat oder Dibutylzinndilaurat. Die erhaltenen Urethanacrylate können einen Gehalt an freien Restmonomeren von unter 0.5 Gew.-%, bevorzugt unter 0.2 Gew.-%, besonders bevorzugt unter 0.1 Gew.-% bezogen auf das Urethanacrylat aufweisen. Die Urethanisierung kann in einem nicht-reaktiven Lösungsmittel, beispielsweise einem aromatischen oder aliphatischen Kohlenwasserstoff oder einem aromatischen oder aliphatischen halogenierten Kohlenwasserstoff oder einem Lacklösungsmittel wie z.B. Ethylacetat oder Butylacetat oder Aceton oder Butanon oder einem Ether wie Tetrahydrofuran oder tert.-Butylmethylether oder einem dipolaraprotischen Lösungsmittel wie Dimethylsulfoxid oder N- Methylpyrrolidon oder N-Ethylpyrrolidon durchgeführt werden.

Die Phenylurethanacrylate gemäß Formel (I) können ferner durch den Zusatz von Stabilisatoren gegen ungewollte Polymerisation geschützt werden. Solche Stabilisatoren können sauerstoffhaltiges Gas wie auch chemische Stabilisatoren, wie sie beispielsweise in Houben-Weyl, Methoden der organischen Chemie, 4. Auflage, Band XIV/1, Georg Thieme Verlag, Stuttgart 1961, Seite 433 ff. beschrieben sind, sein. Beispiele sind: Natriumdithionit, Natriumhydrogensulfid, Schwefel, Hydrazin, Phenylhydrazin, Hydrazobenzol, N-Phenyl-βnaphthylamin, N-Phenyl-ethanoldiamin, Dinitrobenzol, Picrinsäure, p-Nitroso-dimethylanilin, Diphenylnitrosamin, Phenole wie para-Methoxyphenol, 2,5-Di-tert.-Butylhydrochinon, 2,6-Di-tert.-butyl-4-methylphenol, p-tert.-Butyl-brenzcatechin oder 2,5-Di-tert.-amyl-hydrochinon, Tetramethyl-thiuramdisulfid, 2-Mercaptobenzothiazol, Dimethyl-dithiocarbaminsäure-natriumsalz, Phenothiazin, N-Oxyl-Verbindungen wie z.B. 2,2,6,6-Tetramethylpiperidin-N-Oxid (TEMPO) oder eines seiner Derivate. Bevorzugt sind 2,6-Di-tert.-butyl-4-methylphenol und para-Methoxyphenol sowie deren Mischungen. Diese Stabilisatoren werden typischerweise in einer Menge von 0.001 bis 1 Gew.-%, bevorzugt 0.01 bis 0.5 Gew. -% bezogen auf das zu stabilisierende Phenylurethanacrylat eingesetzt.

Geeignete Hydroxyalkylacrylate sind 2-Hydroxyethylacrylat, 2-Hydroxyethylmethacrylat, 2-Hydroxypropylacrylat, 2-Hydroxypropylmethacrylat, 3-Hydroxypropylacrylat, 3-Hydroxypropylmethacrylat, 2-Hydroxyalkylacrylat und 2-Hydroxyalkylmethacrylat mit bis zu 8 C-Atomen in der Alkylgruppe $R^1$ in Formel (I), 4-Hydroxybutylacrylat, 4-Hydroxybutylmethacrylat, 5-Hydroxyhexylacrylat, 5-Hydroxyhexylmethacrylat, 8-Hydroxyoctylacrylat, 8-Hydroxyoctylmethacrylat, 3-Hydroxy-2,2-dimethylpropyl(meth)acrylat und deren Gemische.

Bevorzugt sind 2-Hydroxyethylacrylat, 2-Hydroxyethylmethacrylat, 2-Hydroxypropylacrylat, 2-Hydroxypropylmethacrylat, 4-Hydroxybutylacrylat und 4-Hydroxybutylmethacrylat und deren Gemische. Besonders bevorzugt sind 2-Hydroxyethylacrylat und 2-Hydroxypropylacrylat und deren Gemische.

Die substituierten Phenylisocyanate können aus den entsprechenden aromatischen Aminen hergestellt werden. Gängige Verfahren sind die Phosgenierung des Amins in Lösemittel, die Gasphasenphosgenierung, die Reaktion mit Phenoxycarbonylchlorid und die anschließende Phenolabspaltung, die Reaktion mit einem aromatischen Diisocyanat und die anschließende thermische Spaltung und Abdestillation des substituierten Phenylisocyanats.

Die substituierten Phenylisocyanate können nun Urethanisierung mit den Hydroxyalkyl-(meth)acrylaten zu den erfinderischen Verbindungen der Formel (I) umgesetzt werden.

Geeignete substituierte Phenylisocyanate sind die isomeren Biphenylisocyanate, die isomeren Phenylthiophenylisocyanate, die isomeren Phenylthiobiphenylisocyanate, die isomeren Phenylthiophenylthiophenylisocyanate, die isomeren Bis-(phenylthio)-phenylisocyanate. Weiterhin können die zuvor genannten substituierten Phenylisocyanate zusätzlich noch eine Alkylgruppe mit bis zu 8 C-Atomen und/oder eine Alkylthiogruppe mit bis zu 4 C-Atomen und/oder ein Halogen tragen. Bevorzugte Alkylgruppen als weitere Substituenten sind Methyl, Ethyl, Propyl, Butyl und Hexyl. Bevorzugte Alkylthiogruppen sind die Methylthio und Ethylthiogruppen. Bevorzugte Halogene sind Fluor und Chlor. So sind geeignete Phenylisocyanate z.B. 2-Isocyanato-5-methylbiphenyl, 2-Isocyanato-5-ethylbiphenyl, 2-Isocyanato-5-propylbiphenyl, 2-Isocyanato-5-butylbiphenyl, 2-Isocyanato-5-hexylbiphenyl, 2-Isocyanato-5-octylbiphenyl, 6-Isocyanatobiphenyl-3-yl-methylsulfid, 6-Isocyanatobiphenyl-3-yl-ethyl-sulfid, 6-Isocyanatobiphenyl-3-yl-phenylsulfid, 2-Isocyanato-2'-methylbiphenyl, 2-Iso-cyanato-2'-ethylbiphenyl, 2-Isocyanato-2'-propylbiphenyl, 2-Isocyanato-2'-butylbiphenyl, 2-Fluor-2'-isocyanatobiphenyl, 2-Chlor-2'-isocyanatobiphenyl, 2-Brom-2'-isocyanatobiphenyl, 2-Iod-2'-isocyanatobiphenyl, 2'-Isocyanatobiphenyl-2-yl-methylsulfid, 2'-Isocyanatobiphen-yl-2-yl-ethylsulfid, 2'-Isocyanatobiphenyl-2-yl-phenylsulfid, 5-Fluor-2-isocyanatobiphenyl, 5-Chlor-2-isocyanatobiphenyl, 5-Brom-2-isocyanatobiphenyl, 5-Iod-2-isocyanatobiphenyl, 2-Isocyanato-3,5-dimethylbiphenyl, 2,3,4,5-Tetrafluor-6-isocyanatobiphenyl, 2-Isocyanato-5-methylphenyl-phenylsulfid, 2-Isocyanato-5-ethylphenyl-phenylsulfid, 2-Isocyanato-5-propylphenyl-phenylsulfid, 2-Isocyanato-5-butylphenyl-phenylsulfid, 5-Fluor-2-isocyanatophenyl-phenylsulfid, 5-Chlor-2-isocyanatophenyl-phenylsulfid, 1-Isocyanato-4-(methylsulfanyl)-2-(phenylsulfanyl)benzol, 4-(Ethylsulfanyl)-1-isocyanato-2-(phenylsulfanyl)benzol, 2-Isocyanato-3,5-dimethylphenyl-phenylsulfid, 1-Isocyanato-2-[(2-methylphenyl)sulfanyl]-benzol, 1-Isocyanato-2- [(2-ethylphenyl)sulfanyl]benzol, 1-Isocyanato-2-[(2-propylphenyl)sulfanyl]benzol, 1-Isocyanato-2- [(2-butylphenyl)sulfanyl]benzol, 1-Isocyanato-2-[(2-hexylphenyl)sulfanyl]benzol, 1-Fluor-2-[(2-isocyanatophenyl)sulfanyl]benzol, 1-Chlor-2-[(2-isocyanatophenyl)sulfanyl]benzol, 1-Brom-2-[(2-isocyanatophenyl)sulfanyl]benzol, 1-Iod-2-[(2-isocyanatophenyl)sulfanyl]benzol und deren Regioisomere.

Bevorzugte substituierte Phenylisocyanate sind Biphenylisocyanat, Phenylthiophenylisocyanat, Phenylthiobiphenylisocyanat, Phenylthiophenylthiophenylisocyanat und Bis-(phenylthio)-phenylisocyanat.

Besonders bevorzugte substituierte Phenylisocyanate sind 2-Biphenylisocyanat, 2-Phenylthiophenylisocyanat und 1-Isocyanato-2-{[3-(phenylsulfanyl)phenyl]sulfanyl}benzol.

Geeignet sind auch substituierte Napthylisocyanate, wie z.B. 1-Isocyanato-4-phenylnaphthalin, 4-Isocyanato-5-methyl-1-phenylnaphthalin, 4-Isocyanato-5-ethyl-1-phenylnaphthalin, 4-Isocyanato-5-butyl-1-phenylnaphthalin, 5-Fluor-4-isocyanato-1-phenylnaphthalin, 5-Chlor-4-isocyanato-1-phenylnaphthalin, 5-Brom-4-isocyanato-1-phenylnaphthalin, 5-Iod-4-isocyanato-1-phenylnaphthalin, 4-Isocyanato-5-(methylsulfanyl)-1-phenylnaphthalin, 4-Isocyanato-5-(ethylsulfanyl)-1-phenylnaphthalin,

4-Isocyanato-1-naphthyl-phenylsulfid, 4-Isocyanato-5-methyl-1-naphthyl-phenylsulfid, 4-Isocyanato-5-ethyl-1-naphthyl-phenylsulfid, 4-Isocyanato-5-propyl-1-naphthyl-phenylsulfid, 4-Isocyanato-5-butyl-1-naphthyl-phenylsulfid, 4-Isocyanato-5-octyl-1-naphthyl-phenylsulfid, 5-Fluor-4-isocyanato-1-naphthyl-phenylsulfid, 5-Chlor-4-isocyanato-1-naphthyl-phenylsulfid, 5-Brom-4-isocyanato-1-naphthyl-phenylsulfid, 5-Iod-4-isocyanato-1-naphthylphenylsulfid, 4-Isocyanato-5-(methylsulfanyl)-1-(phenylsulfanyl)naphthalin, 4-Isocyanato-5-(ethylsulfanyl)-1-(phenylsulfanyl)naphthalin sowie deren Regioisomere. Bevorzugt sind 1-Isocyanato-4-phenylnaphthalin und 4-Isocyanato-1-naphthyl-phenylsulfid.

Bei der Verbindung der Formel (I) kann der Rest $R^3$ ein mit einem oder mehreren Phenyl-, Phenythiyl-, Alkyl-, Alkylthiyl-, Halogen, Biphenyl, Naphtyl_Resten substituierter Phenyl- oder Phenylthiyl-Rest sein.

Gemäß einer bevorzugten Ausführungsform ist aber vorgesehen, dass bei der Verbindung der Formel (I) der Reste $R^3$ aus der Gruppe Phenyl, Phenylthiyl, Phenylthiylphenylthiyl, ausgewählt ist.

Ebenfalls bevorzugt ist, wenn bei der Verbindung der Formel (I) Ar ein Rest der Formel (II) ist. Besonders bevorzugt ist auch, bei der Verbindung der Formel (I) Ar ein Rest der Formel (II) ist undbei der Verbindung der Formel (II) n = 1 ist.

Gemäß einer weiteren bevorzugten Ausführungsform der Erfindung kann $R^1$ ein Rest ausgewählt aus der Gruppe $-CH_2-CH_2-$, $-CH_2-CH_2-CH_2-$, $-CH_2-CHCH_3-$, $-CH_2-CH_2-CH_2-CH_2-$ ist.

Bevorzugt ist, wenn die Verbindung der Formel (I) ausgewählt aus der Gruppe 2-[(Biphenyl-2-ylcarbamoyl)oxy]ethyl-acrylat, 2-[(Biphenyl-2-ylcarbamoyl)oxy]ethyl-methacrylat, 2-[(Biphenyl-2-ylcarbamoyl)oxy]propyl-acrylat, 2-[(Biphenyl-2-ylcarbamoyl)oxy]propylmethacrylat, 2-({[2-(Phenylsulfanyl)phenyl]carbamoyl}oxy)ethyl-acrylat, 2-({[2-(Phenylsulfanyl)phenyl]carbamoyl}oxy)ethyl-methacrylat, 2-({[2-(Phenylsulfanyl)phenyl]carbamoyl}-oxy)propyl-acrylat, 2-({ [2-(Phenylsulfanyl)phenyl]carbamoyl}oxy)propyl-methacrylat, 2-{[(2-{[3-(Phenylsulfanyl)phenyl]sulfanyl} phenyl)carbamoyl]oxy}ethyl-acrylat, 2-{ [(2-{ [3-(Phenylsulfanyl)phenyl]sulfanyl}phenyl)carbamoyl]oxy}ethyl-methacrylat, 2-{[(2-{[3-(Phenylsulfanyl)phenyl]sulfanyl}phenyl)carbamoyl]oxy}propyl-acrylat, 2-{ [(2-{ [3-(Phenylsulfanyl)phenyl]sulfanyl}phenyl)carbamoyl]oxy}propyl-methacrylat ist.

Ganz besonders bevorzugt ist, wenn die Verbindung der Formel (I) ausgewählt aus der Gruppe 2-[(Biphenyl-2-ylcarbamoyl)oxy]ethyl-acrylat, 2-[(Biphenyl-2-ylcarbamoyl)oxy]-propyl-acrylat, 2-({[2-(Phenylsulfanyl)phenyl]carbamoyl}oxy)ethyl-acrylat, 2-({[2-(Phenylsulfanyl)phenyl]carbamoyl}oxy)propyl-acrylat, 2-{[(2-{[3-(Phenylsulfanyl)phenyl]sulfanyl}phenyl)carbamoyl]oxy}ethyl-acrylat, 2-{[(2-{[3-(Phenylsulfanyl)phenyl]sulfanyl}phenyl)carbamoyl]oxy }propyl-acrylat ist.

Ein weiterer Gegenstand der vorliegenden Erfindung ist ein Photopolymer umfassend Matrixpolymere, Schreibmonomere und Photoinitiatoren, wobei die Schreibmonomere eine erfindungsgemäße Verbindung gemäß Formel (I) umfassen.

Als Matrixpolymere können amorphe Thermoplaste wie z.B. Polyacrylate, Polymethylmethacrylate oder Copolymere von Methylmethacrylat, Methacrylsäure oder andere Alkylacrylate und Alkylmethacrylate sowie Acrylsäure, wie z.B. Polybutylacrylat, weiterhin Polyvinylacetat und Polyvinylburyrat seine partiell hydrolysierten Derivate wie Polyvinylalkohole sowie Copolymerisate mit Ethylene und/oder weiteren (Meth)acrylaten, Gelatine, Celluloseester und Celluloseether wie Methylcellulose, Celluloseacetobutyrat, Silikone, wie z.B. Polydimethylsilicon, Polyurethane, Polybutadiene und Polyisoprene, sowie Polyethylenoxide, Epoxyharze, insbesondere aliphatische Epoxyharze, Polyamide, Polycarbonate sowie die in US 4994347A und darin zitierten Systeme verwendet werden.

Besonders bevorzugt ist aber, wenn die Matrixpolymere Polyurethane sind.

Besonders bevorzugt ist auch, wenn die Matrixpolymere vernetzt sind. Insbesondere bevorzugt ist dabei, wenn die Matrixpolymere dreidimensional vernetzt sind.

Epoxyharze können kationisch mit sich selbst vernetzt werden. Weiterhin können auch Säure/anhydride, Amine, Hydoxyalkylamide sowie Thiole als Vernetzer eingesetzt werden.

Silicone können sowohl als Einkomponentensysteme durch Kondensation bei Anwesenheit von Wasser (und ggf. unter Broenstedtsäurenkatalyse) oder als zweikomponentige Systeme durch Zugabe von Kieselsäureester oder zinnorganische Verbindungen vernetzt werden. Ebenso ist die Hydrosilylierungen in Vinyl-Silansystemen möglich.

Ungesättigte Verbindungen, wie z.B. Acryloyl funktionelle Polymere oder ungesättigte Ester können mit Aminen oder Thiolen vernetzt werden. Eine kationische Vinyletherpolymerisation ist auch möglich.

Insbesondere bevorzugt ist aber, wenn die Matrixpolymere vernetzt, bevorzugt dreidimensional vernetzt und ganz besonders bevorzugt dreidimensional vernetzte Polyurethane sind. Polyurethan-Matrixpolymere sind insbesondere durch Umsetzung wenigstens einer Polyisocyanat-Komponente a) mit wenigstens einer Isocyanat-reaktiven-Komponente b) erhältlich.

Die Polyisocyanat-Komponente a) umfasst wenigstens eine organische Verbindung mit wenigstens zwei NCO-Gruppen. Bei diesen organischen Verbindungen kann es sich insbesondere um monomere Di- und Triisocyanate, Polyisocyanate und / oder NCO-funktionelle Prepolymere handeln. Die Polyisocyanat-Komponente a) kann auch Mischungen monomerer Di- und Triisocyanate, Polyisocyanate und / oder NCO-funktioneller Prepolymere enthalten oder daraus bestehen.

Als monomere Di- und Triisocyanate können alle dem Fachmann an sich gut bekannten Verbindungen oder deren Mischungen eingesetzt werden. Diese Verbindungen können aromatische, araliphatische, aliphatische oder cycloaliphatische Strukturen aufweisen. In untergeordneten Mengen können die monomeren Di- und Triisocyanate auch Monoisocyanate, d.h. organische Verbindungen mit einer NCO-Gruppe umfassen.

Beispiele für geeignete monomere Di- und Triisocyanate sind 1,4-Butandiisocyanat, 1,5-Pentandiisocyanat, 1,6-Hexandiisocyanat (Hexamethylendiisocyanat, HDI), 2,2,4-Trimethylhexamethylendiisocyanat und / oder 2,4,4-Trimethylhexamethylendiisocyanat (TMDI), Isophorondiisocyanat (IPDI), 1,8-Diisocyanato-4-(isocyanatomethyl)-octan, Bis-(4,4'-isocyanatocyclohexyl)methan und / oder Bis-(2',4-isocyanatocyclohexyl)methan und / oder deren Mischungen beliebigen Isomerengehalts, 1,4-Cyclohexandiisocyanat, die isomeren Bis-(isocyanatomethyl)cyclohexane, 2,4- und / oder 2,6-Diisocyanato-1-methylcyclohexan (Hexahydro-2,4- und / oder 2,6-toluylendiisocyanat, $H_6$-TDI), 1,4-Phenylendiisocyanat, 2,4- und / oder 2,6-Toluylendiisocyanat (TDI), 1,5-Naphthylendiisocyanat (NDI), 2,4'- und / oder 4,4'-Diphenylmethandiisocyanat (MDI), 1,3-Bis(isocyanatomethyl)benzol (XDI) und / oder das analoge 1,4-Isomere oder beliebige Mischungen der vorgenannten Verbindungen.

Geeignete Polyisocyanate sind Verbindungen mit Urethan-, Harnstoff-, Carbodiimid-, Acylharnstoff-, Amid-, Isocyanurat-, Allophanat-, Biuret-, Oxadiazintrion-, Uretdion- und/oder Iminooxadiazindionstrukturen, die aus den vorgenannten Di- oder Triisocyanaten erhältlich sind.

Besonders bevorzugt handelt es sich bei den Polyisocyanaten um oligomerisierte aliphatische und / oder cycloaliphatische Di- oder Triisocyanate, wobei insbesondere die oben stehenden aliphatischen und / oder cycloaliphatischen Di- oder Triisocyanate verwendet werden können.

Ganz besonders bevorzugt sind Polyisocyanate mit Isocyanurat-, Uretdion- und / oder Iminooxadiazindion-Strukturen sowie Biurete basierend auf HDI oder deren Mischungen.

Geeignete Prepolymere enthalten Urethan- und / oder Harnstoff-Gruppen sowie gegebenenfalls weitere durch Modifizierung von NCO-Gruppen entstandene Strukturen wie oben genannt. Derartige Prepolymere sind beispielsweise durch Umsetzung der oben genannten monomeren Di- und Triisocyanate und / oder Polyisocyanaten a1) mit isocyanatreaktiven Verbindungen b1) erhältlich.

Als isocyanatreaktive Verbindungen b1) können Alkohole, Amino oder MercaptoVerbindungen, bevorzugt Alkohole, verwendet werden. Dabei kann es sich insbesondere um Polyole handeln. Ganz besonders bevorzugt können als isocyanatreaktive Verbindung b1) Polyester-, Polyether-, Polycarbonat-, Poly(meth)acrylat- und/oder Polyurethan-Polyole verwendet werden.

Als Polyesterpolyole sind beispielsweise lineare Polyesterdiole oder verzweigte Polyesterpolyole geeignet, die in bekannter Weise durch Umsetzung von aliphatischen, cycloaliphatischen oder aromatischen Di- bzw. Polycarbonsäuren bzw. ihren Anhydriden mit mehrwertigen Alkoholen einer OH-Funktionalität ≥ 2 erhalten werden können. Beispiele für geeignete Di- bzw. Polycarbonsäuren sind mehrwertige Carbonsäuren wie Bernstein-, Adipin-, Kork-, Sebacin-, Decandicarbon-, Phthal-, Terephthal-, Isophthal- Tetrahydrophthal- oder Trimellithsäure sowie Säureanhydride wie Phthal-, Trimellith- oder Bernsteinsäureanhydrid oder deren beliebige Gemische untereinander. Die Polyesterpolyole können auch auf natürlichen Rohstoffen wie Rizinusöl basieren. Es ist ebenfalls möglich, dass die Polyesterpolyole auf Homo- oder Mischpolymerisaten von Lactonen basieren, die bevorzugt durch Anlagerung von Lactonen bzw. Lactongemischen wie Butyrolacton, ε-Caprolacton und / oder Methyl-ε-caprolacton an hydroxyfunktionelle Verbindungen wie mehrwertige Alkohole einer OH-Funktionalität ≥ 2 beispielsweise der nachstehend genannten Art erhalten werden können.

Beispiele für geeignete Alkohole sind alle mehrwertigen Alkohole wie z.B. die $C_2$ - $C_{12}$-Diole, die isomeren Cyclohexandiole, Glycerin oder deren beliebige Gemische untereinander.

Geeignete Polycarbonatpolyole sind in an sich bekannter Weise durch Umsetzung von organischen Carbonaten oder Phosgen mit Diolen oder Diol-Mischungen zugänglich.

Geeignete organische Carbonate sind Dimethyl-, Diethyl- und Diphenylcarbonat.

Geeignete Diole bzw. Mischungen umfassen die an sich im Rahmen der Polyestersegmente genannten mehrwertigen Alkohole einer OH-Funktionalität ≥ 2, bevorzugt Butandiol-1,4, Hexandiol-1,6 und / oder 3-Methylpentandiol. Auch Polyesterpolyole können zu Polycarbonatpolyolen umgearbeitet werden.

Geeignete Polyetherpolyole sind gegebenenfalls blockweise aufgebaute Polyadditionsprodukte cyclischer Ether an OH- oder NH-funktionelle Startermoleküle.

Geeignete cyclische Ether sind beispielsweise Styroloxide, Ethylenoxid, Propylenoxid, Tetrahydrofuran, Butylenoxid, Epichlorhydrin sowie ihre beliebigen Mischungen.

Als Starter können die an sich im Rahmen der Polyesterpolyole genannten mehrwertigen Alkohole einer OH-Funktionalität ≥ 2 sowie primäre oder sekundäre Amine und Aminoalkohole verwendet werden.

Bevorzugte Polyetherpolyole sind solche der vorgenannten Art ausschließlich basierend auf Propylenoxid oder statistische oder Block-Copolymere basierend auf Propylenoxid mit weiteren 1-Alkylenoxiden. Besonders bevorzugt sind Propylenoxid-homopolymere sowie statistische oder Block-Copolymere, die Oxyethylen-, Oxypropylen- und / oder Oxybutyleneinheiten aufweisen, wobei der Anteil der Oxypropyleneinheiten bezogen auf die Gesamtmenge aller Oxyethylen-, Oxypropylen- und Oxybutyleneinheiten mindestens 20 Gew.-%, bevorzugt mindestens 45 Gew.-% ausmacht. Oxypropylen- und Oxybutylen umfasst hierbei alle jeweiligen linearen und verzweigten $C_3$- und $C_4$-Isomere.

Daneben sind als Bestandteile der Polyol-Komponente b1) als polyfunktionelle, isocyanatreaktive Verbindungen auch niedermolekulare, d.h. mit Molekulargewichten ≤ 500 g/mol, kurzkettige, d.h. 2 bis 20 Kohlenstoffatome enthaltende aliphatische, araliphatische oder cycloaliphatische di-, tri- oder polyfunktionelle Alkohole geeignet.

Dies können beispielsweise in Ergänzung zu den oben genannten Verbindungen Neopentylglykol, 2-Ethyl-2-butylpropandiol, Trimethylpentandiol, stellungs-isomere Diethyloctandiole, Cyclohexandiol, 1,4-Cyclohexandimethanol, 1,6-Hexandiol, 1,2- und 1,4-Cyclohexandiol, hydriertes Bisphenol A, 2,2-Bis(4-hydroxy-cyclohexyl)-propan oder 2,2-Dimethyl-3-hydroxypropionsäure, 2,2-dimethyl-3-hydroxypropyl-ester sein. Beispiele geeigneter Triole sind Trimethylolethan, Trimethylolpropan oder Glycerin. Geeignete höherfunktionelle Alkohole sind Di-(trimethylolpropan), Pentaerythrit, Dipenta-erythrit oder Sorbit.

Besonders bevorzugt ist, wenn die Polyolkomponente ein difunktioneller Polyether-, Polyester oder ein Polyether-polyester-block-copolyester oder ein Polyether-Polyester-Blockcopolymer mit primären OH-Funktionen ist.

Es ist ebenfalls möglich, als isocyanatreaktive Verbindungen b1) Amine einzusetzen. Beispiele geeigneter Amine sind Ethylendiamin, Propylendiamin, Diaminocyclohexan, 4,4'-Dicylohexylmethandiamin, Isophorondiamin (IPDA), difunktionelle Polyamine wie z.B. die Jeffamine®, aminterminierte Polymere, insbesondere mit zahlenmittleren Molmassen ≤ 10000 g/Mol. Mischungen der vorgenannten Amine können ebenfalls verwendet werden.

Es ist ebenfalls möglich, als isocyanatreaktive Verbindungen b1) Aminoalkohole einzusetzen. Beispiele geeigneter Aminoalkohole sind die isomeren Aminoethanole, die isomere Aminopropanole die isomeren Aminobutanole und die isomeren Aminohexanole oder deren beliebige Mischungen.

Alle vorgenannten isocyanatreaktiven Verbindungen b1) können untereinander beliebig vermischt werden.

Bevorzugt ist auch, wenn die isocyanatreaktiven Verbindungen b1) eine zahlenmittlere Molmasse von ≥ 200 und ≤ 10000 g/Mol, weiter bevorzugt ≥ 500 und ≤ 8000 g/Mol und ganz besonders bevorzugt ≥ 800 und ≤ 5000 g/Mol aufweisen. Die OH-Funktionalität der Polyole beträgt bevorzugt 1.5 bis 6.0, besonders bevorzugt 1.8 bis 4.0.

Die Prepolymere der Polyisocyanat-Komponente a) können insbesondere einen Restgehalt an freiem monomeren Di- und Triisocyanaten < 1 Gew.-%, besonders bevorzugt < 0.5 Gew.-% und ganz besonders bevorzugt < 0.3 Gew.-% aufweisen.

Es ist gegebenenfalls auch möglich, dass die Polyisocyanat-Komponente a) vollständig oder anteilsmäßig organische Verbindung enthält, deren NCO-Gruppen ganz oder teilweise mit aus der Beschichtungstechnologie bekannten Blockierungsmitteln umgesetzt sind. Beispiel für Blockierungsmittel sind Alkohole, Lactame, Oxime, Malonester, Pyrazole sowie Amine, wie z.B. Butanonoxim, Diisopropylamin, Malonsäurediethylester, Acetessigester, 3,5-Dimethylpyrazol, ε-Caprolactam, oder deren Mischungen.

Besonders bevorzugt ist, wenn die Polyisocyanat-Komponente a) Verbindungen mit aliphatisch gebundenen NCO-Gruppen umfasst, wobei unter aliphatisch gebundenen NCO-Gruppen derartige Gruppen verstanden werden, die an ein primäres C-Atom gebunden sind. Die isocyanatreaktive Komponente b) umfasst bevorzugt wenigstens eine organische Verbindung, die im Mittel wenigstens 1.5 und bevorzugt 2 bis 3 isocyanatreaktive Gruppen aufweist. Im Rahmen der vorliegenden Erfindung werden als isocyanatreaktive Gruppen bevorzugt Hydroxy-, Amino- oder Mercapto-Gruppen angesehen.

Die isocyanatreaktive Komponente kann insbesondere Verbindungen umfassen, die im Zahlenmittel wenigstens 1.5 und bevorzugt 2 bis 3 isocyanatreaktive Gruppen aufweisen.

Geeignete polyfunktionelle, isocyanatreaktive Verbindungen der Komponente b) sind beispielsweise die oben beschriebenen Verbindungen b1).

Ganz besonders bevorzugt ist auch, wenn die Polyurethane auf Polyester-C4-Polyetherpolyolen basieren.

Photoinitiatoren der Komponente sind üblicherweise durch aktinische Strahlung aktivierbare Verbindungen, die eine Polymerisation der Schreibmonomere auslösen können. Bei den Photoinitiatoren kann zwischen unimolekularen (Typ I) und bimolekularen (Typ II) Initiatoren unterschieden werden. Des Weiteren werden sie je nach ihrer chemischen Natur in Photoinitiatoren für radikalische, anionische, kationische oder gemischte Art der Polymerisation unterschieden.

Typ I-Photoinitiatoren (Norrish-Typ-I) für die radikalische Photopolymerisation bilden beim Bestrahlen durch eine unimolekulare Bindungsspaltung freie Radikale. Beispiele für Typ I-Photoinitiatoren sind Triazine, Oxime, Benzoinether, Benzilketale, Bis-imidazole, Aroylphosphinoxide, Sulfonium- und Iodoniumsalze.

Typ II-Photoinitiatoren (Norrish-Typ-II) für die radikalische Polymerisation bestehen aus einem Farbstoff als Sensibilisator und einem Coinitiator und durchlaufen bei der Bestrahlung mit auf den Farbstoff angepasstem Licht eine bimolekulare Reaktion. Zunächst absorbiert der Farbstoff ein Photon und überträgt aus einem angeregten Zustand Energie auf den Coinitiator. Dieser setzt durch Elektronen- oder Protonentransfer oder direkte Wasserstoffabstraktion die polymerisationsauslösenden Radikale frei.

Im Sinne dieser Erfindung werden bevorzugt Typ II-Photoinitiatoren verwendet.

Solche Photoinitiatorsysteme sind prinzipiell in der EP 0 223 587 A beschriebenen und bestehen bevorzugt aus einer Mischung von einem oder mehreren Farbstoffen mit Ammoniumalkylarylborat(en).

Geeignete Farbstoffe, die zusammen mit einem Ammoniumalkylarylborat einen Typ II-Photoinitiator bilden, sind die in der WO 2012062655 beschriebenen kationischen Farbstoffe in Kombination mit den eben dort beschriebenen

Anionen.

Unter kationischen Farbstoffen werden bevorzugt solche der folgenden Klassen verstanden: Acridin-Farbstoffe, Xanthen-Farbstoffe, Thioxanthen-Farbstoffe, Phenazin-Farbstoffe, Phenoxazin-Farbstoffe, Phenothiazin-Farbstoffe, Tri(het)arylmethan-Farbstoffe - insbesondere Diamino- und Triamino(het)arylmethan-Farbstoffe, Mono-, Di-, Tri- und Pentamethincyanin-Farbstoffe, Hemicyanin-Farbstoffe, extern kationische Merocyanin-Farbstoffe, extern kationische Neutrocyanin-Farbstoffe, Nullmethin-Farbstoffe - insbesondere Naphtholactam-Farbstoffe, Streptocyanin-Farbstoffe. Solche Farbstoffe sind beispielsweise in H. Berneth in Ullmann's Encyclopedia of Industrial Chemistry, Azine Dyes, Wiley-VCH Verlag, 2008, H. Berneth in Ullmann's Encyclopedia of Industrial Chemistry, Methine Dyes and Pigments, Wiley-VCH Verlag, 2008, T. Gessner, U. Mayer in Ullmann's Encyclopedia of Industrial Chemistry, Triarylmethane and Diarylmethane Dyes, Wiley-VCH Verlag, 2000 beschrieben.

Besonders bevorzugt sind Phenazin-Farbstoffe, Phenoxazin-Farbstoffe, Phenothiazin-Farbstoffe, Tri(het)arylmethan-Farbstoffe - insbesondere Diamino- und Triamino(het)arylmethan-Farbstoffe, Mono-, Di-, Tri- und Pentamethincyanin-Farbstoffe, Hemicyanin-Farbstoffe, Nullmethin-Farbstoffe - insbesondere Naphtholactam-Farbstoffe, Streptocyanin-Farbstoffe.

Beispiele für kationische Farbstoffe sind Astrazon Orange G, Basic Blue 3, Basic Orange 22, Basic Red 13, Basic Violett 7, Methylenblau, Neu Methylenblau, Azur A, 2,4-Diphenyl-6-(4-methoxyphenyl)pyrylium, Safranin O, Astraphloxin, Brilliant Grün, Kristallviolett, Ethylviolett und Thionin.

Bevorzugte Anionen sind insbesondere $C_8$- bis $C_{25}$-Alkansulfonat, vorzugsweise $C_{13}$- bis $C_{25}$-Alkansulfonat, $C_3$- bis $C_{18}$-Perfluoralkansulfonat, $C_4$- bis $C_{18}$-Perfluoralkansulfonat, das in der Alkylkette mindestens 3 Wasserstoffatome trägt, $C_9$- bis $C_{25}$-Alkanoat, $C_9$- bis $C_{25}$-Alkenoat, $C_8$- bis $C_{25}$-Alkylsulfat, vorzugsweise $C_{13}$- bis $C_{25}$-Alkylsulfat, $C_8$- bis $C_{25}$-Alkenylsulfat, vorzugsweise $C_{13}$- bis $C_{25}$-Alkenylsulfat, $C_3$- bis $C_{18}$-Perfluoralkylsulfat, $C_4$-bis $C_{18}$-Perfluoralkylsulfat, das in der Alkylkette mindestens 3 Wasserstoffatome trägt, Polyethersulfate basierend auf mindestens 4 Äquivalenten Ethylenoxid und/oder Äquivalenten 4 Propylenoxid, Bis-$C_4$- bis $C_{25}$-Alkyl-, $C_5$- bis $C_7$-Cycloalkyl-, $C_3$- bis Cs-Alkenyl- oder $C_7$- bis $C_{11}$-Aralkyl-sulfosuccinat, durch mindestens 8 Fluoratome substituiertes Bis-$C_2$- bis $C_{10}$-alkylsulfosuccinat, $C_8$- bis $C_{25}$-Alkyl-sulfoacetate, durch mindestens einen Rest der Gruppe Halogen, $C_4$- bis $C_{25}$-Alkyl, Perfluor-Ci- bis Cs-Alkyl und/oder $C_1$- bis $C_{12}$-Alkoxycarbonyl substituiertes Benzolsulfonat, ggf. durch Nitro, Cyano, Hydroxy, $C_1$- bis $C_{25}$-Alkyl, $C_1$- bis $C_{12}$-Alkoxy, Amino, $C_1$- bis $C_{12}$-Alkoxycarbonyl oder Chlor substituiertes Naphthalin- oder Biphenylsulfonat, ggf. durch Nitro, Cyano, Hydroxy, $C_1$- bis $C_{25}$-Alkyl, $C_1$- bis $C_{12}$-Alkoxy, $C_1$- bis $C_{12}$-Alkoxycarbonyl oder Chlor substituiertes Benzol-, Naphthalin- oder Biphenyldisulfonat, durch Dinitro, $C_6$- bis $C_{25}$-Alkyl, $C_4$- bis $C_{12}$-Alkoxycarbonyl, Benzoyl, Chlorbenzoyl oder Toluoyl substituiertes Benzoat, das Anion der Naphthalindicarbonsäure, Diphenyletherdisulfonat, sulfonierte oder sulfatierte, ggf. mindestens einfach ungesättigte $C_8$- bis $C_{25}$-Fettsäureester von aliphatischen $C_1$- bis $C_8$-Alkoholen oder Glycerin, Bis-(sulfo-$C_2$- bis $C_6$-alkyl)-$C_3$- bis $C_{12}$-alkandicarbonsäureester, Bis-(sulfo-$C_2$- bis $C_6$-alkyl)-itaconsäureester, (Sulfo-$C_2$- bis $C_6$-alkyl)-$C_6$- bis $C_{18}$-alkancarbonsäureester, (Sulfo-$C_2$- bis $C_6$-alkyl)-acryl- oder methacrylsäureester, ggf. durch bis zu 12 Halogenreste substituiertes Triscatecholphosphat, ein Anion der Gruppe Tetraphenylborat, Cyanotriphenylborat, Tetraphenoxyborat, $C_4$- bis $C_{12}$-Alkyl-triphenylborat, deren Phenyl- oder Phenoxy-Reste durch Halogen, $C_1$- bis $C_4$-Alkyl und/oder $C_1$- bis $C_4$-Alkoxy substituiert sein können, $C_4$- bis $C_{12}$-Alkyl-trinaphthylborat, Tetra-$C_1$- bis $C_{20}$-alkoxyborat, 7,8- oder 7,9-Dicarba-nido-undecaborat(1-) oder (2-), die gegebenenfalls an den B- und/oder C-Atomen durch eine oder zwei $C_1$- bis $C_{12}$-Alkyl- oder Phenyl-Gruppen substituiert sind, Dodecahydro-dicarbadodecaborat(2-) oder B-$C_1$- bis $C_{12}$-Alkyl-C-phenyl-dodecahydro-dicarbadodecaborat(1-) steht, wobei bei mehrwertigen Anionen wie Naphthalindisulfonat $A^-$ für ein Äquivalent dieses Anions steht, und wobei die Alkan- und Alkylgruppen verzweigt sein können und/oder durch Halogen, Cyano, Methoxy, Ethoxy, Methoxycarbonyl oder Ethoxycarbonyl substituiert sein können.

Bevorzugt ist auch, wenn das Anion $A^-$ des Farbstoffs einen AClogP im Bereich von 1 bis 30, besonders bevorzugt im Bereich von 1 bis 12 und insbesondere bevorzugt im Bereich von 1 bis 6,5 aufweist. Der AClogP wird nach J. Comput. Aid. Mol. Des. 2005, 19, 453; Virtual Computational Chemistry Laboratory, http://www.vcclab.org berechnet.

Geeignete Ammoniumalkylarylborate sind beispielsweise (Cunningham et al., RadTech'98 North America UV/EB Conference Proceedings, Chicago, Apr. 19-22, 1998): Tetrabutylammonium Triphenylhexylborat, Tetrabutylammonium Triphenylbutylborat, Tetrabutylammonium Trinapthylhexylborat, Tetrabutylammonium Tris(4-tert.butyl)-phenylbutylborat, Tetrabutylammonium Tris-(3-fluorphenyl)-hexylborat hexylborat ([191726-69-9], CGI 7460, Produkt der BASF SE, Basel, Schweiz), 1-Methyl-3-octylimidazolium Dipentyldiphenylborat und Tetrabutylammonium Tris-(3-chlor-4-methylphenyl)-hexylborat ([1147315-11-4], CGI 909, Produkt der BASF SE, Basel, Schweiz).

Es kann vorteilhaft sein, Gemische dieser Photoinitiatoren einzusetzen. Je nach verwendeter Strahlungsquelle muss Typ und Konzentration an Photoinitiator in dem Fachmann bekannter Weise angepasst werden. Näheres ist zum Beispiel in P. K. T. Oldring (Ed.), Chemistry & Technology of UV & EB Formulations For Coatings, Inks & Paints, Vol. 3, 1991, SITA Technology, London, S. 61 - 328 beschrieben.

Ganz besonders bevorzugt ist, wenn der Photoinitiator eine Kombination von Farbstoffen, deren Absorptionsspektren zumindest teilweise den Spektralbereich von 400 bis 800 nm abdecken, mit wenigstens einem auf die Farbstoffe abgestimmten Coinitiator umfasst.

Bevorzugt ist auch, wenn wenigstens ein für eine Laserlichtfarbe ausgewählt aus blau, grün und rot geeigneter Photoinitiator in der Photopolymer-Formulierung enthalten ist.

Weiter bevorzugt ist auch, wenn die Photopolymer-Formulierung für wenigstens zwei Laserlichtfarben ausgewählt aus blau, grün und rot je einen geeigneten Photoinitiator enthält.

Ganz besonders bevorzugt ist schließlich, wenn die Photopolymer-Formulierung für jede der Laserlichtfarben blau, grün und rot jeweils einen geeigneten Photoinitiator enthält.

Besonders hohe Brechungsindexkontraste können erzielt werden, wenn die Photopolymer-Formulierung als weiteres Schreibmonomer neben der Verbindung der Formel (I) ein acrylat- oder methacrylatfunktionelles Schreibmonomer umfasst. Besonders bevorzugt sind dabei monofunktionelle Schreibmonomere und insbesondere diejenigen monofunktionellen Urethan(meth)acrylate, die in der US 2010/0036013 A1 beschrieben sind.

Geeignete Acrylat-Schreibmonomere sind insbesondere Verbindungen der allgemeinen Formel (IV)

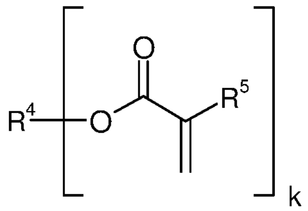

(IV)

bei denen k≥1 und k≤4 ist und $R^4$ ein linearer, verzweigter, cyclischer oder heterocyclischer unsubstituierter oder gegebenenfalls auch mit Heteroatomen substituierter organischer Rest und/oder $R^5$ Wasserstoff, ein linearer, verzweigter, cyclischer oder heterocyclischer unsubstituierter oder gegebenenfalls auch mit Heteroatomen substituierter organischer Rest ist. Besonders bevorzugt ist $R^5$ Wasserstoff oder Methyl und/oder $R^4$ ein linearer, verzweigter, cyclischer oder heterocyclischer unsubstituierter oder gegebenenfalls auch mit Heteroatomen substituierter organischer Rest.

Als Acrylate bzw. Methacrylate werden vorliegend Ester der Acrylsäure bzw. Methacrylsäure bezeichnet. Beispiele bevorzugt verwendbarer Acrylate und Methacrylate sind Phenylacrylat, Phenylmethacrylat, Phenoxyethylacrylat, Phenoxyethylmethacrylat, Phenoxyethoxyethylacrylat, Phenoxyethoxyethylmethacrylat, Phenylthioethylacrylat, Phenylthioethylmethacrylat, 2-Naphthylacrylat, 2-Naphthylmethacrylat, 1,4-Bis-(2-thionaphthyl)-2-butylacrylat, 1,4-Bis-(2-thionaphthyl)-2-butylmethacrylat, Bisphenol A Diacrylat, Bisphenol A Dimethacrylat, sowie deren ethoxylierte Analogverbindungen, N-Carbazolylacrylate.

Als Urethanacrylate werden vorliegend Verbindungen mit mindestens einer Acrylsäureestergruppe und mindestens eine Urethanbindung verstanden. Solche Verbindungen können beispielsweise durch Umsetzung eines Hydroxy-funktionellen Acrylats oder Methacrylats mit einer Isocyanat-funktionellen Verbindung erhalten werden.

Beispiele hierfür verwendbarer Isocyanat-funktionelle Verbindungen sind Monoisocyanate sowie die unter a) genannten monomeren Diisocyanate, Triisocyanate und / oder Polyisocyanate. Beispiele geeigneter Monoisocyanate sind Phenylisocyanat, die isomeren Methylthiophenylisocyanate. Di-, Tri- oder Polyisocyanate sind oben genannt sowie Triphenylmethan-4,4',4"-triisocyanat und Tris(p-isocyanatophenyl)thiophosphat oder deren Derivate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur und Mischungen derselben. Bevorzugt sind dabei aromatische Di-, Tri- oder Polyisocyanate.

Als hydroxyfunktionelle Acrylate oder Methacrylate für die Herstellung von Urethanacrylaten kommen beispielsweise Verbindungen wie 2-Hydroxyethyl(meth)acrylat, Polyethylenoxid-mono(meth)acrylate, Polypropylenoxidmono(meth)acrylate, Polyalkylen-oxidmono(meth)-acrylate, Po|y(ε-caprolacton)mono(meth)acrylate, wie z.B. Tone® M100 (Dow, Schwalbach, DE), 2-Hydroxypropyl(meth)acrylat, 4-Hydroxybutyl(meth)acrylat, 3-Hydroxy-2,2-dimethylpropyl-(meth)acrylat, Hydroxypropyl(meth)acrylat, Acrylsäure-(2-hydroxy-3-phenoxypropylester), die hydroxyfunktionellen Mono-, Di- oder Tetraacrylate mehrwertiger Alkohole wie Trimethylolpropan, Glycerin, Pentaerythrit, Dipentaerythrit, ethoxyliertes, propoxyliertes oder alkoxyliertes Trimethylolpropan, Glycerin, Pentaerythrit, Dipentaerythrit oder deren technische Gemische. Bevorzugt sind 2-Hydroxyethylacrylat, Hydroxypropylacrylat, 4-Hydroxybutylacrylat und Poly(ε-caprolacton)mono(meth)acrylat.

Ebenfalls verwendet werden können die an sich bekannten hydroxylgruppenhaltigen Epoxy-(meth)acrylate mit OH-Gehalten von 20 bis 300 mg KOH/g oder hydroxylgruppenhaltige Polyurethan(meth)acrylate mit OH-Gehalten von 20 bis 300 mg KOH/g oder acrylierte Polyacrylate mit OH-Gehalten von 20 bis 300 mg KOH/g sowie deren Mischungen untereinander und Mischungen mit hydroxylgruppenhaltigen ungesättigten Polyestern sowie Mischungen mit Polyester(meth)acrylaten oder Mischungen hydroxylgruppenhaltiger ungesättigter Polyester mit Polyester(meth)acrylaten.

Bevorzugt sind insbesondere Urethanacrylate erhältlich aus der Umsetzung von Tris(p-isocyanatophenyl)thiophosphat und / oder m-Methylthiophenylisocyanat mit alkoholfunktionellen Acrylaten wie Hydroxyethyl(meth)acrylat, Hydroxypropyl(meth)acrylat und / oder Hydroxybutyl(meth)acrylat.

Ebenso ist es möglich, dass das Schreibmonomer weitere ungesättigte Verbindungen wie α,β-ungesättigte Carbonsäurederivate wie beispielsweise Maleinate, Fumarate, Maleimide, Acrylamide, weiterhin Vinylether, Propenylether, Allylether und Dicyclopentadienyl-Einheiten enthaltende Verbindungen sowie olefinisch ungesättigte Verbindungen wie z.B. Styrol, α-Methylstyrol, Vinyltoluol und / oder Olefine, umfasst oder daraus besteht.

Besonders bevorzugt ist, wenn die Fluorurethane wenigstens eine Verbindung der Formel (V)

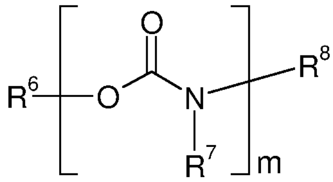

(V)

umfassen oder daraus bestehen, in der $m \geq 1$ und $m \leq 8$ ist und $R^6$, $R^7$, $R^8$ unabhängig voneinander Wasserstoff, lineare, verzweigte, cyclische oder heterocyclische unsubstituierte oder gegebenenfalls auch mit Heteroatomen substituierte organische Reste sind, wobei bevorzugt mindestens einer der Reste $R^6$, $R^7$, $R^8$ mit wenigstens einem Fluoratom substituiert ist und besonders bevorzugt $R^6$ ein organischer Rest mit mindestens einem Fluoratom ist.

Gemäß einer weiteren bevorzugten Ausführungsform der Erfindung ist vorgesehen, dass das Photopolymer 10 bis 89,999 Gew.-%, bevorzugt 20 bis 70 Gew.-% Matrixpolymere, 3 bis 60 Gew.-%, bevorzugt 10 bis 50 Gew.-% Schreibmonomere, 0,001 bis 5 Gew.-%, bevorzugt 0,5 bis 3 Gew.-% Photoinitiatoren und gegebenenfalls 0 bis 4 Gew.-%, bevorzugt 0 bis 2 Gew.-% Katalysatoren, 0 bis 5 Gew.-% , bevorzugt 0,001 bis 1 Gew.-% Stabilisatoren, 0 bis 40 Gew.-%, bevorzugt 10 bis 30 Gew.-% monomere Fluorurethane und 0 bis 5 Gew.-%, bevorzugt 0.1 bis 5 Gew.-% weitere Additive enthält, wobei die Summe aller Bestandteile 100 Gew.-% beträgt.

Besonders bevorzugt werden Photopolymer mit 20 bis 70 Gew.-% Matrixpolymeren, 20 bis 50 Gew.-% Schreibmonomere, 0,001 bis 5 Gew.-% Photoinitiatoren, 0 bis 2 Gew.-% Katalysatoren, 0,001 bis 1 Gew.-% Radikalstabilisatoren gegebenenfalls 10 bis 30 Gew.-% Fluorurethane und gegebenenfalls 0.1 bis 5 Gew.-% weiterer Additive eingesetzt.

Als Katalysatoren können Urethanisierungskatalysatoren, wie z.B. organische oder anorganischen Derivate des Bimuths, des Zinns, des Zinks oder des Eisens (siehe dazu auch die in der US 2012/062658 genannten Verbindungen) verwendet werden. Besonders bevorzugte Katalysatoren sind Butylzinn-tris(2-ethylhexanoat), Eisen(III) - trisacetylacetonat, Bismuth(III) tris(2-ethylhexanoat), und Zinn(II) bis(2-ethylhexanoat). Weiterhin können auch sterisch gehinderte Amine als Katalysatoren eingesetzt werden.

Als Stabilisatoren können Radikalinhibitoren wie HALS-Amine, N-Alkyl-HALS-, N-Alkoxy-HALS- und N-Alkoxyethyl-HALS-Verbindungen sowie Antioxidantien und / oder UV Absorber zum Einsatz kommen.

Als weitere Additive können Verlaufshilfsmittel und / oder Antistatika und / oder Thixotropiermittel und / oder Verdicker und / oder Biozide eingesetzt werden.

Ein weiterer Gegenstand der Erfindung ist ein holographisches Medium, dass das erfindungsgemäße Photopolymer umfasst oder daraus besteht.

Das holographische Medium kann insbesondere ein Films sein, der bevorzugt eine Filmdicke 0.3 µm bis 500 µm, weiter bevorzugt von 0.5 µm bis 200 µm und besonders bevorzugt von 1 µm bis 100 µm aufweisen kann.

Bei einer bevorzugten Ausführungsform des erfindungsgemäßen holographisches Mediums ist in dieses wenigstens ein Hologramm einbelichtet.

Insbesondere kann das Hologramm ein Reflektions-, Transmissions-, In-Line-, Off-Axis-, Full-Aperture Transfer-, Weißlicht-Transmissions-, Denisyuk-, Off-Axis Reflektions- oder Edge-Lit Hologramm sowie ein holographisches Stereogramm und bevorzugt ein Reflektions-, Transmissions- oder Edge-Lit Hologramm sein.

Mögliche optische Funktionen der Hologramme entsprechen den optische Funktionen von Lichtelementen wie Linsen, Spiegel, Umlenkspiegel, Filter, Streuscheiben, gerichteten Streuelementen, Beugungselemente, Lichtleiter, Lichtlenker (waveguides), Projektionsscheiben und/oder Masken. Zudem können mehrere derartiger optischer Funktionen in einem solchen Hologramm kombiniert werden, z.B. so dass je nach Lichteinfall das Licht in eine andere Richtung abgebeugt wird. So kann man beispielweise mit derartigen Aufbauten autostereoskopische oder holographische elektronische Displays bauen, die es erlauben einen stereoskopischen visuellen Eindruck ohne weitere Hilfsmittel wie z.B. einer Polarisator- oder Shutterbrille zu erleben, der Verwendung in automobilen Head-up Displays oder Head-mounted Displays.

Häufig zeigen diese optischen Elemente eine spezifische Frequenzselektivität, je nachdem wie die Hologramme

belichtet wurden und welche Dimensionen das Hologramm hat. Dies ist insbesondere wichtig, wenn man monochromatische Lichtquellen wie LED oder Laserlicht verwendet. So benötigt man ein Hologramm pro Komplementärfarbe (RGB), um Licht frequenzselektiv zu lenken und gleichzeitig vollfarbige Displays zu ermöglichen. Daher sind in bestimmten Displayaufbauten mehrer Hologramme ineinander im Medium zu belichten.

Zudem können mittels der erfindungsgemäßen Medien auch holographische Bilder oder Darstellungen, wie zum Beispiel für persönliche Portraits, biometrische Darstellungen in Sicherheitsdokumenten, oder allgemein von Bilder oder Bildstrukturen für Werbung, Sicherheitslabels, Markenschutz, Markenbranding, Etiketten, Designelementen, Dekorationen, Illustrationen, Sammelkarten, Bilder und dergleichen sowie Bilder, die digitale Daten repräsentieren können u.a. auch in Kombination mit den zuvor dargestellten Produkten hergestellt werden. Holographische Bilder können den Eindruck eines dreidimensionalen Bildes haben, sie können aber auch Bildsequenzen, kurze Filme oder eine Anzahl von verschiedenen Objekten darstellen, je nachdem aus welchem Winkel, mit welcher (auch bewegten) Lichtquelle etc. diese beleuchtet wird. Aufgrund dieser vielfältigen Designmöglichkeiten stellen Hologramme, insbesondere Volumenhologramme, eine attraktive technische Lösung für die oben genannten Anwendung dar. Auch ist es möglich derartige Hologramme zur Speicherung digitaler Daten zu verwenden, wobei verschiedenste Belichtungsverfahren (Shift-, Spatial- oder Angular- Multiplexing) verwendet werden.

Gegenstand der vorliegenden Erfindung ist auch noch ein Verfahren zur Herstellung eines holographischen Mediums unter Verwendung einer erfindungsgemäßen Photopolymer-Formulierung.

So können die Photopolymere insbesondere zur Herstellung holographischer Medien in Form eines Films verwendet werden. Dabei wird als Träger eine Lage eines für Licht im sichtbaren Spektralbereich (Transmission größer als 85% im Wellenlängenbereich von 400 bis 780 nm) transparenten Materials oder Materialverbunds ein- oder beidseitig beschichtet sowie ggf. eine Abdeckschicht auf der oder den Photopolymerlagen appliziert.

Bevorzugte Materialien oder Materialverbünde des Trägers basieren auf Polycarbonat (PC), Polyethylenterephthalat (PET), Polybutylenterephthalat, Polyethylen, Polypropylen, Celluloseacetat, Cellulosehydrat, Cellulosenitrat, Cycloolefinpolymere, Polystyrol, Polyepoxide, Polysulfon, Cellulosetriacetat (CTA), Polyamid (PA), Polymethylmethacrylat (PMMA), Polyvinylchlorid, Polyvinylbutyral oder Polydicyclopentadien oder deren Mischungen. Besonders bevorzugt basieren sie auf PC, PET, PA, PMMA und CTA. Materialverbünde können Folienlaminate oder Coextrudate sein. Bevorzugte Materialverbünde sind Duplex- und Triplexfolien aufgebaut nach einem der Schemata A/B, A/B/A oder A/B/C. Besonders bevorzugt sind PC/PET, PET/PC/PET und PC/TPU (TPU = Thermoplastisches Polyurethan).

Die Materialien oder Materialverbünde des Trägers können einseitig oder beidseitig antihaftend, antistatisch, hydrophobiert oder hydrophiliert ausgerüstet sein. Die genannten Modifikationen dienen an der Photopolymerschicht zugewandten Seite dem Zweck, dass die Photopolymerlage von dem Träger zerstörungsfrei abgelöst werden kann. Eine Modifikation der der Photopolymerlage abgewandten Seite des Trägers dient dazu, dass die erfindungsgemäßen Medien speziellen mechanischen Anforderungen genügen, die z.B. bei der Verarbeitung in Rollenlaminatoren, insbesondere bei Rolle-zu-Rolle-Verfahren, gefordert sind.

Ebenfalls Gegenstand der Erfindung ist eine optische Anzeige, umfassend ein erfindungsgemäßes holographisches Medium.

Beispiele für derartige optische Anzeigen sind bildgebende Anzeigen auf Basis von Flüssigkristallen, organischen lichtemittierenden Dioden (OLED), LED-Displaytafeln, Microelektromechanische Systeme (MEMS) auf Basis von diffraktiver Lichtselektion, Electrowettingdisplays (E-ink) und Plasmabildschirmen. Derartige optische Anzeigen können autostereoskopische und/oder holographische Displays, transmittive und reflektive Projektionsleinwände oder Projektionsscheiben, Displays mit schaltbaren eingeschränkten Abstrahlverhalten für Privacyfilter und bidirektionalen Multiuserbildschirmen, virtuelle Bildschirme, Head-up-Displays, Head-mounted Displays, Leuchtsymbole, Warnlampen, Signallampen, Scheinwerfer und Schautafeln sein.

Ebenfalls Gegenstand der Erfindung sind autostereoskopische und/oder holographische Displays, Projektionsleinwände, Projektionsscheiben, Displays mit schaltbaren eingeschränkten Abstrahlverhalten für Privacyfilter und bidirektionalen Multiuserbildschirmen, virtuelle Bildschirme, Headup-Displays, Head-mounted Displays, Leuchtsymbole, Warnlampen, Signallampen, Scheinwerfer und Schautafeln umfassend ein erfindungsgemäßes holographisches Medium.

Noch weitere Gegenstände der Erfindung sind ein Sicherheitsdokument und ein holographisch optisches Element umfassend ein erfindungsgemäßes holographisches Medium.

Darüber hinaus ist auch die Verwendung eines erfindungsgemäßen holographischen Mediums zur Herstellung von Chipkarten, Ausweisdokumenten, 3D-Bildern, Produktschutzetiketten, Labeln, Banknoten oder holographisch optischen Elementen insbesondere für optische Anzeigen Gegenstand der Erfindung.

Weiterhin Gegenstand der Erfindung ist die Verwendung der Verbindung der Formel (I) als Schreibmonomer in Photopolymeren, holographischen Medien und/oder holographisch optischen Elementen.

## Beispiele

Die Erfindung wird im Folgenden anhand von Beispielen näher erläutert.

In der Zeichnung zeigt:

Figur 1 die Geometrie eines Holographic Media Testers (HMT) bei $\lambda$ = 532 nm (DPSS Laser = Diode Pumped Solid State Laser).

Figur 2 die gemessene Beugungseffizienz $\eta$ als Kreise gegen das Winkeldetuning $\Delta\Omega$ aufgetragen und die Anpassung der Kogelnik Theorie als durchgezogene Linie. Die Darstellung zeigt Beispiel 2.

Figur 3 die gemessene Beugungseffizienz $\eta$ als Kreise gegen das Winkeldetuning $\Delta\Omega$ aufgetragen und die Anpassung der Kogelnik Theorie als durchgezogene Linie. Die Darstellung zeigt Beispiel 4.

Figur einen Aufbau zum Schreiben von Denisiyuk-Hologrammen.

## Messmethoden:

### Viskositätsbestimmung:

Die Viskosität wurde mit einem Physica MCR 51 (Fa. Anton Paar) Viskosimeter bestimmt, Dazu wurde temperiert und ein Kegel eingehangen (für niedrige Viskositäten $\eta$ < 10000 mPas: 23°C, 25 mm (CP-25) Kegeldurchmesser und für hohe Viskositäten $\eta$ > 10000 mPas 50°C, 60 mm (CP-60) Kegeldurchmesser). Man gab ca. 0.5-1 g Produkt auf die Platte, ließ den Kegel herunter fahren, so daß der Kegel mit Produkt vollständig benetzt war. Überstehendes Produkt wurde abgewischt. Die Scherrate (ca. 500 1/s bei niedrigen Viskositäten und ca. 100 1/s bei höheren Viskositäten) wurde von dem Gerät selbständig eingestellt. Es wurden jeweils 20 Messwerte gemessen und der Mittelwert bestimmt.

### Isocyanatgehalt

Die angegebenen NCO-Werte (Isocyanat-Gehalte) wurden gemäß DIN EN ISO 11909 bestimmt.

Der vollständige Umsatz von NCO-Gruppen bzw. deren Abwesenheit in einem Reaktionsgemisch wurde IR-spektroskopisch nachgewiesen. So wurde dann ein vollständiger Umsatz angenommen, wenn im IR-Spektrum des Reaktionsgemisches keine NCO-Bande (2261cm$^{-1}$) sichtbar war.

### Messung der holographischen Eigenschaften Beugungseffizienz DE und Brechungsindexkontrast $\Delta$n der holographischen Medien mittels Zweistrahlinterferenz in Reflexionsanordnung.

Mit einem holographischen Versuchsaufbau wie in Figur 1 dargestellt, wurden die Beugungseffizienz (DE) der Medien gemessen. Der Strahl eines DPSS Lasers (Emissionswellenlänge 532 nm) wurde mit Hilfe des Raumfilter (SF) und zusammen mit der Kollimationslinse (CL) in einen parallelen homogenen Strahl umgewandelt. Die finalen Querschnitte des Signal und Referenzstrahls werden durch die Irisblenden (I) festgelegt. Der Durchmesser der Irisblendenöffnung beträgt 0.4 cm. Die polarisationsabhängigen Strahlteiler (PBS) teilen den Laserstrahl in zwei kohärente gleich polarisierte Strahlen. Über die $\lambda/2$ Plättchen wurden die Leistung des Referenzstrahls auf 0.87 mW und die Leistung des Signalstrahls auf 1.13 mW eingestellt. Die Leistungen wurden mit den Halbleiterdetektoren (D) bei ausgebauter Probe bestimmt. Der Einfallswinkel ($\alpha_0$) des Referenzstrahls beträgt -21.8°, der Einfallswinkel (ßo) des Signalstrahls beträgt 41.8°. Die Winkel werden ausgehend von der Probennormale zur Strahlrichtung gemessen. Gemäß Figur 1 hat daher $\alpha_0$ ein negatives Vorzeichen und $\beta_0$ ein positives Vorzeichen. Am Ort der Probe (Medium) erzeugte das Interferenzfeld der zwei überlappenden Strahlen ein Gitter heller und dunkler Streifen die senkrecht zur Winkelhalbierenden der zwei auf die Probe einfallenden Strahlen liegen (Reflexionshologramm). Der Streifenabstand A, auch Gitterperiode genannt, im Medium beträgt -225 nm (der Brechungsindex des Mediums zu ~1.504 angenommen).

Figur 1 zeigt die Geometrie eines Holographic Media Testers (HMT) bei $\lambda$ = 532 nm (DPSS Laser): M = Spiegel, S = Verschluss, SF = Raumfilter, CL = Kollimatorlinse, $\lambda/2$ = $\lambda/2$ Platte, PBS = polarisationsempfindlicher Strahlteiler, D = Detektor, I = Irisblende, $\alpha_0$ = -21.8°, $\beta_0$ = 41.8° sind die Einfallswinkel der kohärenten Strahlen außerhalb der Probe (des Mediums) gemessen. RD = Referenzrichtung des Drehtisches.

Es wurden auf folgende Weise Hologramme in das Medium geschrieben:

- Beide Shutter (S) sind für die Belichtungszeit t geöffnet.

- Danach wurde bei geschlossenen Shuttern (S) dem Medium 5 Minuten Zeit für die Diffusion der noch nicht polymerisierten Schreibmonomere gelassen.

Die geschriebenen Hologramme wurden nun auf folgende Weise ausgelesen. Der Shutter des Signalstrahls blieb geschlossen. Der Shutter des Referenzstrahls war geöffnet. Die Irisblende des Referenzstrahls wurde auf einen Durchmesser < 1 mm geschlossen. Damit erreichte man, dass für alle Drehwinkel ($\Omega$) des Mediums der Strahl immer vollständig im zuvor geschriebenen Hologramm lag. Der Drehtisch überstrich nun computergesteuert den Winkelbereich von $\Omega_{min}$ bis $\Omega_{max}$ mit einer Winkelschrittweite von 0.05°. $\Omega$ wird von der Probennormale zur Referenzrichtung des Drehtisches gemessen. Die Referenzrichtung des Drehtisches ergibt sich dann wenn beim Schreiben des Hologramms der Einfallswinkel des Referenz- und des Signalstrahls betragsmäßig gleich sind also $\alpha_0$ = -31.8° und $\beta_0$ = 31.8° gilt. Dann beträgt $\Omega_{recording}$ = 0°. Für $\alpha_0$ = -21.8° und $\beta_0$ = 41.8° beträgt $\Omega_{recording}$ daher 10°. Allgemein gilt für das Interferenzfeld beim Schreiben ("recording") des Hologramms:

$$\alpha_0 = \theta_0 + \Omega_{recording} .$$

$\theta_0$ ist der Halbwinkel im Laborsystem außerhalb des Mediums und es gilt beim Schreiben des Hologramms:

$$\theta_0 = \frac{\alpha_0 - \beta_0}{2} .$$

In diesem Fall gilt also $\theta_0$ = -31.8°. An jedem angefahrenen Drehwinkel $\Omega$ wurden die Leistungen des in der nullten Ordnung transmittierten Strahls mittels des entsprechenden Detektors D und die Leistungen des in die erste Ordnung abgebeugten Strahls mittels des Detektors D gemessen. Die Beugungseffizienz ergab sich bei jedem angefahrenen Winkel $\Omega$ als der Quotient aus:

$$\eta = \frac{P_D}{P_D + P_T}$$

$P_D$ ist die Leistung im Detektor des abgebeugten Strahls und $P_T$ ist die Leistung im Detektor des transmittierten Strahls.

Mittels des oben beschriebenen Verfahrens wurde die Braggkurve, sie beschreibt den Beugungswirkungsgrad $\eta$ in Abhängigkeit des Drehwinkels $\Omega$ des geschriebenen Hologramms gemessen und in einem Computer gespeichert. Zusätzlich wurde auch die in die nullte Ordnung transmittierte Intensität gegen den Drehwinkel $\Omega$ aufgezeichnet und in einem Computer gespeichert.

Die maximale Beugungseffizienz (DE = $\eta_{max}$) des Hologramms, also sein Spitzenwert, wurde bei $\Omega_{reconstruction}$ ermittelt. Eventuell musste dazu die Position des Detektors des abgebeugten Strahls verändert werden, um diesen maximalen Wert zu bestimmen.

Der Brechungsindexkontrast $\Delta$n und die Dicke d der Photopolymerschicht wurde nun mittels der Coupled Wave Theorie (siehe; H. Kogelnik, The Bell System Technical Journal, Volume 48, November 1969, Number 9 Seite 2909 - Seite 2947) an die gemessene Braggkurve und den Winkelverlauf der transmittierten Intensität ermittelt. Dabei ist zu beachten, dass wegen der durch die Photopolymerisation auftretenden Dickenschwindung der Streifenabstand $\Lambda'$ des Hologramms und die Orientierung der Streifen (slant) vom Streifenabstand $\Lambda$ des Interferenzmusters und dessen Orientierung abweichen kann. Demnach wird auch der Winkel $\alpha_0'$ bzw. der entsprechende Winkel des Drehtisches $\Omega_{reconstruction}$, bei dem maximale Beugungseffizienz erreicht wird von $\alpha_0$ bzw. vom entsprechenden $\Omega_{recording}$ abweichen. Dadurch verändert sich die Bragg-Bedingung. Diese Veränderung wird im Auswerteverfahren berücksichtigt. Das Auswerteverfahren wird im Folgenden beschrieben:

Alle geometrischen Größen, die sich auf das geschriebene Hologramm beziehen und nicht auf das Interferenzmuster werden als gestrichene Größen dargestellt.

Für die Braggkurve $\eta(\Omega)$ eines Reflexionshologramms gilt nach Kogelnik:

$$\eta = \begin{cases} \dfrac{1}{1 - \dfrac{1-(\xi/\nu)^2}{\sin^2\left(\sqrt{\xi^2-\nu^2}\right)}}, \text{für } \nu^2 - \xi^2 < 0 \\ \dfrac{1}{1 + \dfrac{1-(\xi/\nu)^2}{\sinh^2\left(\sqrt{\nu^2-\xi^2}\right)}}, \text{für } \nu^2 - \xi^2 \geq 0 \end{cases}$$

mit:

$$\nu = \frac{\pi \cdot \Delta n \cdot d'}{\lambda \cdot \sqrt{|c_s \cdot c_r|}}$$

$$\xi = -\frac{d'}{2 \cdot c_s} \cdot DP$$

$$c_s = \cos(\vartheta') - \cos(\psi') \cdot \frac{\lambda}{n \cdot \Lambda'}$$

$$c_r = \cos(\vartheta')$$

$$DP = \frac{\pi}{\Lambda'} \cdot \left(2 \cdot \cos(\psi' - \vartheta') - \frac{\lambda}{n \cdot \Lambda'}\right)$$

$$\psi' = \frac{\beta' + \alpha'}{2}$$

$$\Lambda' = \frac{\lambda}{2 \cdot n \cdot \cos(\psi' - \alpha')}$$

Beim Auslesen des Hologramms ("reconstruction") gilt wie analog oben dargestellt:

$$\vartheta'_0 = \theta_0 + \Omega$$

$$\sin(\vartheta'_0) = n \cdot \sin(\vartheta')$$

An der Bragg-Bedingung ist das "Dephasing" *DP* = 0. Und es folgt entsprechend:

$$\alpha'_0 = \theta_0 + \Omega_{reconstruction}$$

$$\sin(\alpha'_0) = n \cdot \sin(\alpha')$$

Der noch unbekannt Winkel $\beta'$ kann aus dem Vergleich der Bragg-Bedingung des Interferenzfeldes beim Schreiben des Hologramms und der Bragg-Bedingung beim Auslesen des Hologramms ermittelt werden unter der Annahme, dass nur Dickenschwindung stattfindet. Dann folgt:

$$\sin(\beta') = \frac{1}{n} \cdot \left[\sin(\alpha_0) + \sin(\beta_0) - \sin(\theta_0 + \Omega_{reconstruction})\right]$$

v ist die Gitterstärke, $\xi$ ist der Detuning Parameter und $\psi'$ die Orientierung (Slant) des Brechungsindexgitters das geschrieben wurde. $\alpha'$ und $\beta'$ entsprechen den Winkeln $\alpha_0$ und $\beta_0$ des Interferenzfeldes beim Schreiben des Hologramms, aber im Medium gemessen und für das Gitter des Hologramms gültig (nach Dickenschwindung). n ist der mittlere Brechungsindex des Photopolymers und wurde zu 1.504 gesetzt. $\lambda$ ist die Wellenlänge des Laserlichts im Vakuum.

Die maximale Beugungseffizienz (DE = $\eta_{max}$) ergibt sich dann für $\xi$ = 0 zu:

$$DE = \tanh^2(\nu) = \tanh^2\left(\frac{\pi \cdot \Delta n \cdot d'}{\lambda \cdot \sqrt{\cos(\alpha') \cdot \cos(\alpha' - 2\psi)}}\right)$$

Die Figuren 2 und 3 zeigt die gemessene transmittierte Leistung $P_T$ (rechte *y*-Achse) als durchgezogene Linie gegen das Winkeldetuning $\Delta\Omega$ aufgetragen, die gemessene Beugungseffizienz $\eta$ (linke *y*-Achse) als ausgefüllte Kreise gegen das Winkeldetuning $\Delta\Omega$ aufgetragen (soweit die endliche Größe des Detektors es erlaubte) und die Anpassung der Kogelnik Theorie als gestrichelte Linie (linke *y*-Achse).

Die Messdaten der Beugungseffizienz, die theoretische Braggkurve und die transmittierte Intensität werden wie in Figur 2 und 3 gezeigt gegen den zentrierten Drehwinkel $\Delta\Omega = \Omega_{reconstruction} - \Omega = (\alpha'_0 - \vartheta'_0$, auch Winkeldetuning genannt, aufgetragen.

Da DE bekannt ist wird die Form der theoretischen Braggkurve nach Kogelnik nur noch durch die Dicke *d'* der Photopolymerschicht bestimmt. $\Delta$n wird über DE für gegebene Dicke *d'* so nachkorrigiert, dass Messung und Theorie von DE immer übereinstimmen. *d'* wird nun solange angepasst bis die Winkelpositionen der ersten Nebenminima der theoretischen Braggkurve mit den Winkelpositionen der ersten Nebenmaxima der transmittierten Intensität übereinstimmen und zudem die volle Breite bei halber Höhe (FWHM) für die theoretische Braggkurve und für die transmittierte Intensität übereinstimmen.

Da die Richtung in der ein Reflexionshologramm bei der Rekonstruktion mittels eines $\Omega$-Scans mitrotiert, der Detektor für das abgebeugte Licht aber nur einen endlichen Winkelbereich erfassen kann, wird die Braggkurve von breiten Holgrammen (kleines *d'*) bei einem $\Omega$-Scan nicht vollständig erfasst, sondern nur der zentrale Bereich, bei geeigneter Detektorpositionierung. Daher wird die zur Braggkurve komplementäre Form der transmittierten Intensität zur Anpassung der Schichtdicke *d'* zusätzlich herangezogen.

Die Figuren 2 und 3 zeigen die Darstellung der Braggkurve $\eta$ nach der Coupled Wave Theorie (gestrichelte Linie), des gemessenen Beugungswirkungsgrades (ausgefüllte Kreise) und der transmittierten Leistung (schwarz durchgezogene Linie) gegen das Winkeldetuning $\Delta\Omega$.

Für eine Formulierung wurde diese Prozedur eventuell mehrfach für verschiedene Belichtungszeiten t an verschiedenen Medien wiederholt, um festzustellen bei welcher mittleren Energiedosis des einfallenden Laserstrahls beim Schreiben des Hologramms DE in den Sättigungswert übergeht. Die mittlere Energiedosis E ergibt sich wie folgt aus den Leistungen der zwei den Winkeln $\alpha_0$ und $\beta_0$ zugeordneten Teilstrahlen (Referenzstrahl mit $P_r$ = 0.87 mW und Signalstrahl mit $P_s$ = 1.13 mW), der Belichtungszeit t und dem Durchmesser der Irisblende (0.4 cm):

$$E\ (\mathrm{mJ/cm^2}) = \frac{2 \cdot [P_r + P_s] \cdot t\ (\mathrm{s})}{\pi \cdot 0.4^2\ \mathrm{cm}^2}$$

Die Leistungen der Teilstrahlen wurden so angepasst, dass in dem Medium bei den verwendeten Winkeln $\alpha_0$ und $\beta_0$, die gleiche Leistungsdichte erreicht wird.

## Chemikalien:

In eckigen Klammern ist jeweils, soweit bekannt die CAS-Nummer angegeben.

| | |
|---|---|
| 2-Hydroxyethylacrylat | [818-61-1] - Sigma-Aldrich Chemie GmbH Steinheim, Deutschland |
| Hydroxypropylacrylat | [25584-83-2] - BASF SE, Ludwigshafen Deutschland |
| 2,6-Di-tert.-butyl-4-methylphenol | [128-37-0] - Merck KGaA, Darmstadt, Germany |
| 2-Aminobiphenyl | [90-41-5] - Sigma-Aldrich Chemie GmbH Steinheim, Deutschland |

(fortgesetzt)

| | |
|---|---|
| 2-Aminobiphenylphenylsulfid | [1134-94-7]-Sigma-Aldrich Chemie GmbH Steinheim, Deutschland |
| 3-(Methylthio)phenylisocyanat | [28479-19-8] - Sigma-Aldrich Chemie GmbH Steinheim, Deutschland |
| 1-Isocyanato-3-(methylsulfanyl)benzol | [28479-19-8] - Sigma-Aldrich Chemie GmbH, Steinheim, Deutschland |
| Desmodur® RFE | Tris(p-isocyanatophenyl)thiophosphat, 27%-ig in Ethylacetat, Produkt der Bayer MaterialScience AG, Leverkusen, Deutschland |
| Dibutylzinndilaurat | [77-58-7]-Sigma-Aldrich Chemie GmbH Steinheim, Deutschland |
| Fomrez® UL 28 | Momentive Performance Chemicals, Wilton, CT, USA. |
| Borchi® Kat 22 | [85203-81-2] - OMG Borchers GmbH, Langenfeld, Deutschland. |
| BYK-310 | BYK-Chemie GmbH, Wesel, Deutschland |
| Chlorameisensäurephenylester | [1885-14-9]-Acros Organics, Geel, Belgium |
| Desmodur® N 3900 | Bayer MaterialScience AG, Leverkusen, DE, Hexandiisocyanatbasiertes Polyisocyanat, Anteil an Iminooxadiazindion mindestens 30 %, NCO-Gehalt: 23.5 %. |
| Desmodur 2460M | Bayer MaterialScience AG, Leverkusen, DE, Bis-(Isocyanatophenyl) methan (MDI) basiertes Isocyanat. |
| Desmorapid® SO | [301-10-0] - Rhein Chemie Rheinau GmbH, Mannheim, Deutschland |
| CGI-909 | Tetrabutylammonium-tris(3-chlor-4-methylphenyl)-(hexyl)borat, [1147315-11-4], BASF SE |
| Trimethylhexamethylendiisocyanat | [28679-16-5] - ABCR GmbH & Co KG, Karlsruhe, Deutschland |
| 1H,1H-7H-Perfluorheptan-1-ol | [335-99-9] - ABCR GmbH & Co KG, Karlsruhe, Deutschland |
| Astrazon Rosa FG 200% | [3648-36-0] - DyStar Colours Deutschland GmbH, Frankfurt am Main, Deutschland |
| Natrium-bis(2-ethylhexyl)sulfosuccinat | [45297-26-5] Sigma-Aldrich Chemie GmbH, Steinheim, Deutschland |

**Herstellung von 2-Phenylthiophenylisocyanat**

In einer Dreihalsapparatur mit KPG Rührer wurden unter Stickstoff 720 g 2-Aminobiphenylphenylsulfid in 4.2 kg Toluol gelöst und 519 g Kaliumkarbonat unter Rühren hinzugegeben und auf 10 °C temperiert. Nun tropfte man 560 g Chlorameisensäurephenylester zu. Das Produkt wurde abfiltriert und im Vakuum getrocknet. Man erhielt 1.15 kg Phenyl-[2-(phenylsulfanyl)phenyl]carbamat in Form eines kristallinen Niederschlags.

950 g Phenyl-[2-(phenylsulfanyl)phenyl]carbamat wurden in einem Dreihalskolben vorgelegt, der mit einem KPG-Rührwerk, einer verspiegelten Vigreuxkolonne und Destillationsbrücke versehen war. Es wurde ein Vakuum von ca 1 mbar angelegt und langsam auf 168°C erhitzt. Bei einer Kopftemperatur von 143°C wurden zunächst 257 g Phenol abdestilliert. Danach erhielt man insgesamt 592 g Rohprodukt mit einem NCO-Gehalt von 16.5%. Das Rohprodukt wurde bei 1 mbar und 118-121 °C Kopftemperatur feindestilliert und man erhielt insgesamt 502 g 2-Phenylthiophenylisocyanat.

**Herstellung von 2-Biphenylisocyanat**

In einem Dreihalskolben mit Tropftrichter, KPG-Rührwerk und Destillationsaufsatz wurden 1500 g Desmodur 2460M vorgelegt und auf 140°C erwärmt. Nun gab man innerhalb von 50 Minuten 243.7 g 2-Aminobiphenyl zu und hielt die Reaktionstemperatur unter 160 °C. Anschließend wurde das Produkt im Hochvakuum (ca. 0.03 mbar) abdestilliert und man erhielt 203.2 g 2-Biphenylisocyanat einer klaren Flüssigkeit erhalten.

**Herstellung von 1-Isocyanato-2-{[3-(phenylsulfanyl)phenyl]sulfanyl}benzol**

100 g 2-{[3-(Phenylsulfanyl)phenyl]sulfanyl}anilin (hergestellt wie in Advanced Synthesis & Catalysis (2009), 351(14+15), 2369-2378 beschrieben) und 0.1 g Tetrabutylammoniumbromid wurden in 470 g Dichlormethan/ Wasser (1:1) gelöst und mit 49.1 g Kaliumcarbonat versetzt. In die intensiv gerührte Mischung wurden bei 10 °C 53.1 g Chlorameisensäurephenylester getropft. Nach Reaktionsende wurden 0.52 g Methanol zugegeben und eine weitere Stunde bei Raumtemperatur gerührt. Das Rohprodukt wurde auf 1 L Wasser ausgetragen und die wäßrige Phase wurde dreimal mit je 500 mL Dichlormethan extrahiert. Die organischen Phasen wurden getrocknet und das Lösungsmittel wurde im Vakuum abdestilliert. Man erhielt 133 g Phenyl-(2-{[3-(phenylsulfanyl)phenyl]sulfanyl}phenyl)carbamat als farblosen Feststoff.

100 g Phenyl-(2-{[3-(phenylsulfanyl)phenyl]sulfanyl}phenyl)carbamat wurden in einer Kugelrohr-Destillationsapparatur vorgelegt und bei 160°C und 0.1 mbar erhitzt. Das entstandene Phenol wurde verworfen und man erhielt 72 g Rohprodukt. Nach erneuter Destillation in einer Kugelrohr-Destillationsapparatur wurden 65.3 g 1-Isocyanato-2-{[3-(phenylsulfanyl)-phenyl]sulfanyl}benzol als farblose Flüssigkeit erhalten.

**Beispiel 1: 2-({[2-(Phenylsulfanyl)phenyl]carbamoyl}oxy)propyl-acrylat**

In einem Dreihalskolben mit Kühler, KPG-Rührwerk und Stickstoffgaseinlass wurden 16.0 g 2-Phenylthiophenylisocyanat vorgelegt und das Reaktionsgefäß mit Stickstoff gespült und danach auf 80 °C erwärmt. Nun gab man 5 mg 2,6-Di-tert.-butyl-4-methylphenol und 1 mg Bochi-Kat 22 zu. Nach 15 Minuten Rühren gab man innerhalb von 20 Minuten 9.01 g Hydroxypropylacrylat tropfenweise hinzu. Man rührte 18 Stunden lang und erhielt 2-({[2-(Phenylsulfanyl)phenyl]carbamoyl}oxy)propyl-acrylat als klare Flüssigkeit, die kein Isocyanat mehr enthielt.

**Beispiel 2: 2-({[2-(Phenylsulfanyl)phenyl]carbamoyl}oxy)ethyl-acrylat**

In einem Dreihalskolben mit Kühler, KPG-Rührwerk und Lufteinlass wurden 45.9 g 2-Phenylthiophenylisocyanat, 13.5 mg 2,6-Di-tert.-butyl-4-methylphenol und 33.8 mg Dibutylzinndilaurat vorgelegt danach auf 60 °C erwärmt. Nach 20 Minuten Rühren gab man innerhalb von 10 Minuten 21.6 g Hydroxyethylacrylat tropfenweise hinzu. Man rührte 19 Stunden lang und gab weiter 30 mg Dibutylzinndilaurat hinzu. Nach weiteren 32 Stunden wurden weitere 1.08 g Hydroxyethylacrylat zugegeben und man erhielt nach weiteren 5 Stunden Rühren 2-({[2-(Phenylsulfanyl)phenyl]carbamoyl}oxy)ethyl-acrylat als eine klare Flüssigkeit, die kein Isocyanat mehr enthielt.

**Beispiel 3: 2-[(Biphenyl-2-ylcarbamoyl)oxy]propyl-acrylat**

In einem Dreihalskolben mit Kühler, KPG-Rührwerk und Lufteinlass wurden 7.8 g 2-Biphenylisocyanat und 2.6 mg 2,6-Di-tert.-butyl-4-methylphenol vorgelegt und danach auf 60 °C erwärmt. Nun gab man unter langsamer Lufteinleitung innerhalb von 30 Minuten 5.2 g Hydroxypropylacrylat tropfenweise hinzu. Nach 1.5 Stunden gab man 6.5 mg Dibutylzinndilaurat hinzu. Man rührte 46 Stunden lang und erhielt 2-[(Biphenyl-2-ylcarbamoyl)oxy]propyl-acrylat als eine klare Flüssigkeit, die kein Isocyanat mehr enthielt.

**Beispiel 4: 2-[(Biphenyl-2-ylcarbamoyl)oxy]ethyl-acrylat**

In einem Dreihalskolben mit Kühler, KPG-Rührwerk und Lufteinlass wurden 7.8 g 2-Biphenylisocyanat und 2.4 mg 2,6-Di-tert.-butyl-4-methylphenol vorgelegt und danach auf 60 °C erwärmt. Nun gab man unter langsamer Lufteinleitung innerhalb von 30 Minuten 4.3 g 2-Hydroxyethylacrylat tropfenweise hinzu. Nach 1.5 Stunden gab man 6.1 mg Dibutylzinndilaurat hinzu. Man rührte 70 Stunden lang und erhielt 2-[(Biphenyl-2-ylcarbamoyl)oxy]-ethyl-acrylat als klare Flüssigkeit, die kein Isocyanat mehr enthielt und langsam zu einem Feststoff mit einem Schmelzbereich von 110-120°C kristallisierte.

**Beispiel 5: 2-{[(2-{[3-(Phenylsulfanyl)phenyl]sulfanyl}phenyl)carbamoyl]oxy}ethyl-acrylat**

In einem 100 mL Rundkolben wurden 0.01 g 2,6-Di-tert.-butyl-4-methylphenol, 0.01 g Dibutylzinndilaurat sowie und 7.50 g 1-Isocyanato-2-{[3-(phenylsulfanyl)phenyl]sulfanyl}-benzol in 30 mL Ethylacetat vorgelegt und auf 60 °C erwärmt. Anschließend wurden 2.50 g 2-Hydroxyethylacrylat zugetropft und die Mischung weiter auf 60 °C gehalten, bis der Isocyanatgehalt unter 0.1 % gesunken war. Danach wurde abgekühlt und im Vakuum das Ethylacetat vollständig entfernt. Das Produkt wurde als teilkristalliner Feststoff erhalten.

**Beispiel 6: 2-{[(2-{[3-(Phenylsulfanyl)phenyl]sulfanyl}phenyl)carbamoyl]oxy}propyl-acrylat**

In einem 100 mL Rundkolben wurden 0.01 g 2,6-Di-tert.-butyl-4-methylphenol, 0.01 g Dibutylzinndilaurat sowie und 7.30 g 1-Isocyanato-2-{[3-(phenylsulfanyl)phenyl]sulfanyl}-benzol in 30 mL Ethylacetat vorgelegt und auf 60 °C erwärmt. Anschließend wurden 2.70 g Hydroxypropylacrylat zugetropft und die Mischung weiter auf 60 °C gehalten, bis der Isocyanatgehalt unter 0.1 % gesunken war. Danach wurde abgekühlt und im Vakuum das Ethylacetat vollständig entfernt. Das Produkt wurde als teilkristalliner Feststoff erhalten.

**Vergleichsbeispiel A: 2-({[3-(Methylsulfanyl)phenyl]carbamoyl}oxy)ethylprop-2-enoat**

In einem 100 mL Rundkolben wurden 0.02 g 2,6-Di-tert.-butyl-4-methylphenol, 0.01 g Dibutylzinndilaurat, 11.7 g 3-(Methylthio)phenylisocyanat vorgelegt und auf 60 °C erwärmt. Anschließend wurden 8.2 g 2-Hydroxyethylacrylat

zugetropft und die Mischung weiter auf 60 °C gehalten, bis der Isocyanatgehalt unter 0.1 % gesunken war. Danach wurde abgekühlt. Das Produkt wurde als farblose Flüssigkeit erhalten.

**Urethanacrylat 1: Phosphorothioyltris(oxybenzol-4,1-diylcarbamoyloxyethan-2,1-diyl)trisacrylat**

In einem 500 mL Rundkolben wurden 0.1 g 2,6-Di-tert.-butyl-4-methylphenol, 0.05 g Dibutylzinndilaurat sowie und 213.1 g einer 27 %-igen Lösung von Tris(p-isocyanatophenyl)-thiophosphat in Ethylacetat (Desmodur® RFE, Produkt der Bayer MaterialScience AG, Leverkusen, Deutschland) vorgelegt und auf 60 °C erwärmt. Anschließend wurden 42.4 g 2-Hydroxyethylacrylat zugetropft und die Mischung weiter auf 60 °C gehalten, bis der Isocyanatgehalt unter 0.1 % gesunken war. Danach wurde abgekühlt und im Vakuum das Ethylacetat vollständig entfernt. Das Produkt wurde als teilkristalliner Feststoff erhalten.

**Polyol-Komponente:**

In einem 1 L Kolben wurden 0.037 g Desmorapid® SO, 374.8 g ε-Caprolacton und 374.8 g eines difunktionellen Polytetrahydrofuranpolyetherpolyols vorgelegt und auf 120 °C aufgeheizt und so lange auf dieser Temperatur gehalten, bis der Festgehalt (Anteil der nichtflüchtigen Bestandteile) bei 99.5 Gew.-% oder darüber lag. Anschließend wurde abgekühlt und das Produkt als wachsiger Feststoff erhalten.

**Farbstoff 1:**

5.84 g wasserfreies Natrium-bis(2-ethylhexyl)sulfosuccinat wurden in 75 mL Essigsäureehylester gelöst. 14.5 g des Farbstoffs Astrazon Rosa FG 200%, gelöst in 50 mL Wasser, wurden zugesetzt. Die wässrige Phase wurde abgetrennt und die organische Phase wurde dreimal mit 50 ml frischem Wasser bei 50 °C verrührt und jedes Mal die wässrige Phase abgetrennt, die letzte bei Raumtemperatur. Nach Abtrennen der wässrigen Phase wurde das Lösungsmittels im Vakuum abdestilliert und man erhielt 8.6 g 3H-Indolium, 2-[2-[4-[(2-chloroethyl)methylamino]phenyl]ethenyl]-1,3,3-trimethyl-1,4-bis(2-ethylhexyl) sulfosuccinat [153952-28-4] als hochviskoses Öl.

**Fluoriertes Urethan: Bis(2,2,3,3,4,4,5,5,6,6,7,7-dodecafluoroheptyl)-(2,2,4-trimethyl-hexane-1,6-diyl)biscarbamat**

In einem 6 L-Rundkolben wurden 0.50 g Desmorapid Z und 1200 g Trimethylhexamethylendiisocyanat vorgelegt und auf 80 °C erwärmt. Anschließend wurden 3798 g 1H,1H,7H-Perfluorheptan-1-ol zugetropft und die Mischung weiter auf 80 °C gehalten, bis der Isocyanatgehalt unter 0.1 % gesunken war. Danach wurde abgekühlt. Das Produkt wurde als farbloses Öl erhalten.

**Herstellung der erfindungsgemäßen und nicht erfindungsgemäßen holographischen Medien (Beispiele I-VI und Vergleichbeispiel I)**

7.90 g der oben beschriebenen Polyol-Komponente wurden aufgeschmolzen und mit 7.65 g des jeweiligen Schreibmonomers (Schreibmonomer 1 bis 6 sowie Vergleichsbeispiel A), 2.57 g des oben beschriebenen Urethanacrylats 1, 5.10 g des oben beschriebenen fluorierten Urethans, 0.91 g CGI 909, 0.232 g des Farbstoff 1, 0.230 g BYK 310, 0.128 g Fomrez UL 28 und 3.789 g Ethylacetat gemischt, so dass eine klare Lösung erhalten wurde. Anschließend wurden 1.50 g Desmodur® N 3900 zugegeben und erneut gemischt.

Nun gab man diese Lösung in einer Rolle zu Rolle Beschichtungsanlage auf eine 36 µm dicke PET Folie, wo mittels eines Rakels das Produkt in einer Naßschichtdicke von 19 µm appliziert wurde. Bei einer Trocknungstemperatur von 85 °C und einer Trocknungszeit von 5 Minuten wurde die beschichtete Folie getrocknet und anschließend mit einer 40 µm dicken Polyethylenfolie geschützt. Anschließend wurde dieser Film lichtdicht verpackt.

**Bestimmung der Feuchtigkeitsstabilität der Hologramme, die in den erfindungsgemäßen und nicht erfindungsgemäßen Medien aufgezeichnet wurden.**

Die wie im Abschnitt "Herstellung der erfindungsgemäßen und nicht erfindungsgemäßen holographischen Medien" beschrieben hergestellten Medien wurden anschließend mittels einer Messanordnung gemäß Figur 4 wie folgt auf ihre holographischen Eigenschaften geprüft:
Der Strahl eines Lasers (Emissionswellenlänge 532 nm) wird mit Hilfe einer optionalen Aufweitungslinse (AF) und der Kollimationslinse (CL), die nach dem Shutter S platziert ist auf einen Durchmesser von ~ 3 - 4 cm aufgeweitet. Der Durchmesser des aufgeweiteten Laserstrahls wird dabei durch die Apertur des geöffneten Shutters bestimmt. Es wird

bewusst auf eine inhomogene Intensitätsverteilung des aufgeweiteten Laserstrahles geachtet. So beträgt die Randintensität $P_R$ ~ nur die Hälfte der Intensität $P_Z$ im Zentrum des aufgeweiteten Laserstrahls. P ist hier als Leistung/Fläche zu verstehen. Der aufgeweitete Laserstrahl durchläuft zunächst eine schräg zum Strahl gestellte Glasplatte, die als Shearing Plate (SP) dient. Anhand des nach oben gespiegelten Interferenzmusters, das von den zwei Glasoberflächenreflexen der SP erzeugt wird, kann erkannt werden, ob der Laser stabil im Single Mode emittiert. Dann ist auf einer über der SP platziert Mattscheibe aus dunklen und hellen Streifen zu sehen. Nur wenn Single Mode Emission gegeben ist werden holographischen Belichtungen durchgeführt. Im Falle der DPSS Laser kann der Single Mode durch einstellen des Pumpstroms erreicht werden. Der aufgeweitet Strahl durchläuft das etwa 15° schräg gestellte holographische Medium (P), dieser Teil bildet den Referenzstrahl, um dann vom zu P parallel angeordneten Objekt (O) wieder zurück in P reflektiert zu werden. Dieser Teil bildet dann den Signalstrahl der Denisyukanordnung.

Die Interferenz von Signal- und Referenzstrahl in P erzeugt das Hologramm im holographischen Medium. O besteht aus einer mit weißem Papier bedeckten Metallplatte, wobei die Papierseite P zugewandt ist. Auf dem Papier befindet sich ein quadratisches Raster erzeugt durch schwarze Linien. Die Kantenlänge eines Quadrates beträgt 0.5 cm. Dieses Raster wird bei der holographischen Belichtung von P mit im Hologramm abgebildet.

Die mittlere Belichtungsdosis $E_{ave}$ wird durch die Öffnungszeit *t* von S eingestellt. Bei fixierter Laserleistung I stellt *t* daher die zu $E_{ave}$ proportionale Größe dar. Da der aufgeweitete Laserstrahl eine inhomogenene (glockenförmige) Intensitätsverteilung besitzt variiert die lokale Dosis E zur Erzeugung des Hologramms in P. Dies führt, zusammen mit der Schrägstellung von P und O zur optischen Achse dazu, dass das geschriebene Hologramm eine elliptische Form besitzt. Da es sich bei O um einen diffusen Reflektor handelt ist das Hologramm durch Beleuchten mit einer Punktlichtquelle (z. B. Taschenlampe) leicht zu rekonstruieren und es ist ebenfalls möglich, die Hologramme im Transmission-Modus eines UV-VIS Spektrometers zu untersuchen und miteinander zu vergleichen.

Anschließend wurden die Proben mit der Substratseite zur Lampe hin auf das Förderband eines UV-Strahlers gelegt und mit einer Bahngeschwindigkeit von 2,5 m/min zwei Mal belichtet. Als UV-Strahler wurde eine eisendotierte Hg-Lampe vom Typ Fusion UV type "D Bulb" No. 558434 KR 85 mit 80 W / $cm^2$ Gesamtleistungsdichte verwendet. Die Parameter entsprachen einer Dosis von 2 x 2,0 J/$cm^2$ (gemessen mit einem Light Bug des Typs ILT 490).

Die so erhaltenen Medien wurde in einem UV-VIS Spektrometer untersucht. Dazu wurde eine Transmissionsmessung durch das Medium durchgeführt und aufgezeichnet. Über die Auswertung der Transmissionskurve läßt sich die geringste Transmission bestimmen, dies entspricht der höchsten Beugungseffizienz. Nun wird die Resonanzfrequenz (in nm) im Transmissionspektrum bei geringster Transmission bestimmt und als $T_{min}$ angegeben.

Im Folgenden wurde die Transmissionspektren der geschriebenen Hologramme der erfindungsgemäßen Beispiele 1-6 und dem Vergleichsbeispiel A bestimmt. Die Medien enthaltend die wie oben beschrieben hergestellten Hologramme wurden unter verschiedenen Temperatur- und Luftfeuchtigkeiten gelagert und $T_{min}$ wurde nach Abschluss der Lagerung erneut bestimmt.

Untersuchung der Temperaturstabilität:

Die Proben wurden für zwei Tage bei 100 °C in einem Ofen gelagert, innerhalb von 2 Minuten abgekühlt und $T_{min}(1)$ bestimmt. Anschließend wurden die Proben für 7 Tage bei ca 20°C und 40-50% relative Feuchte gelagert und $T_{min}(2)$ bestimmt. Schließlich wurde die Differenz der Peakwellenlängen der beiden Messungen $\Delta T_{min}(1)$ errechnet.

Untersuchung der Feuchtestabilität:

Die Proben wurden für zwei Tage bei 60 °C/95 % relativer Luftfeuchtigkeit gelagert, innerhalb von 2 Minuten abgekühlt und $T_{min}(3)$ bestimmt. Anschließend wurden die Proben für 7 Tage bei ca 20 °C und 40-50% relative Feuchte gelagert und $T_{min}(4)$ bestimmt. Schließlich wurde die Differenz der Peakwellenlängen der beiden Messungen $\Delta T_{min}(2)$ errechnet.

Untersuchung der holographischen Performance (siehe Tabelle 1)

Die bestimmung der Brechungsindex-Modulation $\Delta n$ wurde gemäß dem oben im Abschnitt "Messmethoden" beschriebenen Verfahren durchgeführt. Die erfindungsgemäßen Beispiele 1-6 und das Vergleichsbeispiel A zeigen gute holographische Performance mit einer Brechungsindex-Modulation $\Delta n > 0.025$. Bei der Untersuchung der thermischen Stabilität wurde durchgehend ein Wert von < 2 nm $\Delta T_{min}(1)$ gefunden. Bei der Untersuchung der Feuchtestabilität wurde für die erfindungsgemäßen Beispiele durchgehend ein Wert von < 5 nm $\Delta T_{min}(2)$ gefunden. Das Vergleichsbeispiel weist ein $\Delta T_{min}(2)$ von 7.4 nm auf. Somit weisen die erfindungsgemäßen Beispiele der Formel (I) eine maximale Veränderung der Rekonstruktionswellenlänge von weniger als 5 nm bezogen auf ein Reflektionshologramm, das durch Interferenz zweier ebener Wellen mit einer Wellenlänge von 532 nm geschrieben wurde, auf.

**Tabelle 1:**

| Beispiel | $\Delta n$ | $T_{min}$ | $T_{min}(1)$ | $T_{min}(2)$ | $\Delta T_{min}(1)$ | $T_{min}(3)$ | $T_{min}(4)$ | $\Delta T_{min}(2)$ |
|---|---|---|---|---|---|---|---|---|
| Einheit | - | [nm] | [nm] | [nm] | [nm] | [nm] | [nm] | [nm] |
| 1 | 0.029 | 530.7 | 529.0 | 530.0 | -1.0 | 534.8 | 530.7 | 4.1 |
| 2 | 0.033 | 529.3 | 527.8 | 528.4 | -0.6 | 533.1 | 528.4 | 4.7 |
| 3 | 0.028 | 529.0 | 527.4 | 527.8 | -0.4 | 532.3 | 527.8 | 4.5 |
| 4 | 0.027 | 527.1 | 525.3 | 525.9 | -0.6 | 531.5 | 526.7 | 4.8 |
| 5 | 0.034 | 530.7 | 528.2 | 530.0 | -1.9 | 533.5 | 529.8 | 3.7 |
| 6 | 0.036 | 529.7 | 527.2 | 528.6 | -1.4 | 532.9 | 529.6 | 3.3 |
| Vergleichsbeispiel | | | | | | | | |
| A | 0.033 | 526.9 | 524.5 | 525.1 | -0.6 | 532.7 | 525.3 | 7.4 |

## Patentansprüche

**1.** Photopolymer umfassend Matrixpolymere, Schreibmonomere, monomere Fluorurethane und Photoinitiatoren, wobei die Schreibmonomere Verbindung der Formel (I)

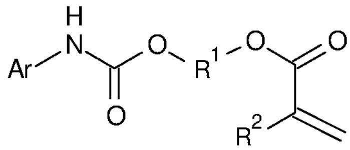

(I);

in der

$R^1$ ein aliphatischer Kohlenwasserstoffrest mit 1-8 C-Atomen ist;
$R^2$ Wasserstoff oder Methyl ist;
Ar ein aromatischer Rest der Formel (II) ist

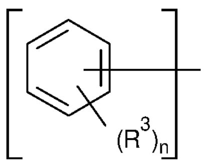

(II),

in der

$R^3$ unabhängig voneinander Reste ausgewählt aus der Gruppe substituiertes oder unsubstituiertes Phenyl, substituiertes oder unsubstituiertes Phenylthiyl, verzweigtes oder unverzweigtes Alkyl, verzweigtes oder unverzweigtes Alkylthiyl, Halogen sind, wobei wenigstens einer der Reste $R^3$ ein Rest ausgewählt aus der Gruppe substituiertes oder unsubstituiertes Phenyl, substituiertes oder unsubstituiertes Phenylthiyl ist;
n = 1 bis 5 ist;

oder Ar ein aromatischer Rest der Formel (III) ist

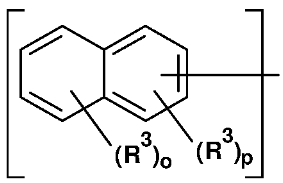

(III),

in der

$R^3$ unabhängig voneinander Reste ausgewählt aus der Gruppe substituiertes oder unsubstituiertes Phenyl, substituiertes oder unsubstituiertes Phenylthiyl, verzweigtes oder unverzweigtes Alkyl, verzweigtes oder unverzweigtes Alkylthiyl, Halogen sind, wobei wenigstens einer der Reste $R^3$ ein Rest ausgewählt aus der Gruppe substituiertes oder unsubstituiertes Phenyl, substituiertes oder unsubstituiertes Phenylthiyl ist.
o = 1 bis 3;
p = 1 bis 4 ist,

wobei die Verbindung der Formel (I) nur eine strahlenhärtende Gruppe aufweist, umfassen.

2. Photopolymer gemäß Anspruch 1, dadurch gekennzeichnte, dass wenigstens einer der Reste $R^3$ ausgewählt aus der Gruppe Phenyl, Phenylthiyl, Phenylthiylphenylthiyl, Alkylphenyl, Alkylphenylthiyl, Biphenyl, ist.

3. Photopolymer nach einem der Ansprüche 1 oder 2, **dadurch gekennzeichnet, dass** Ar ein Rest der Formel (II) ist.

4. Photopolymer nach einem der Ansprüche 1 bis 3, **dadurch gekennzeichnet, dass** o = 1 ist.

5. Photopolymer nach einem der Ansprüche 1 bis 4, **dadurch gekennzeichnet, dass** $R^1$ ein Rest ausgewählt aus der Gruppe $-CH_2-CH_2-$, $-CH_2-CH_2-CH_2-$, $-CH_2-CHCH_3-$,$-CH_2-CH_2-CH_2-CH_2-$ ist.

6. Photopolymer nach Anspruch 1-5, **dadurch gekennzeichnet, dass** die Matrixpolymere vernetzt, bevorzugt dreidimensional vernetzt und ganz besonders bevorzugt dreidimensional vernetzte Polyurethane sind.

7. Holographisches Medium umfassend ein Photopolymer gemäß einem der Ansprüche 1 bis 6.

8. Holographisches Medium nach Anspruch 7, **dadurch gekennzeichnet, dass** es ein Films ist, der bevorzugt eine Filmdicke 0.3 µm bis 500 µm, weiter bevorzugt von 0.5 µm bis 200 µm und besonders bevorzugt von 1 µm bis 100 µm aufweisen kann.

9. Holographisches Medium nach einem der Ansprüche 7 bis 8, **dadurch gekennzeichnet, dass** wenigstens ein Hologramm einbelichtet ist.

10. Optische Anzeige umfassend ein holographisches Medium nach einem der Ansprüche 7 bis 9.

11. Sicherheitsdokument umfassend ein holographisches Medium nach einem der Ansprüche 7 bis 9.

12. Holographisch optisches Element, umfassend ein holographisches Medium nach einem der Ansprüche 7 bis 9.

13. Verbindung der Formel (I')

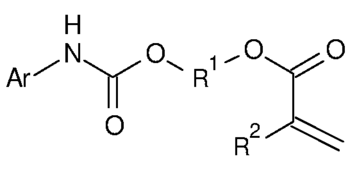

(I');

in der

$R^1$ ein aliphatischer Kohlenwasserstoffrest mit 1-8 C-Atomen ist;
$R^2$ Wasserstoff oder Methyl ist;
Ar ein aromatischer Rest der Formel (II') ist

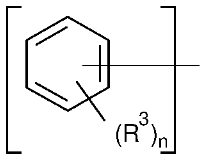

(II'),

in der

$R^{3'}$ unabhängig voneinander Reste ausgewählt aus der Gruppe unsubstituiertes Phenyl, substituiertes oder unsubstituiertes Phenylthiyl, verzweigtes oder unverzweigtes Alkyl, verzweigtes oder unverzweigtes Alkylthiyl, Halogen sind, wobei wenigstens einer der Reste $R^{3'}$ ein Rest ausgewählt aus der Gruppe unsubstituiertes Phenyl, substituiertes oder unsubstituiertes Phenylthiyl ist;
n = 1 bis 5 ist;

oder Ar ein aromatischer Rest der Formel (III) ist

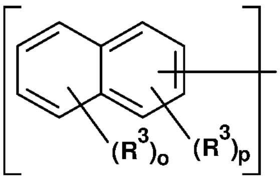

(III),

in der

$R^3$ ein mit einem oder mehreren Phenyl-, Phenythiyl-, Alkyl-, Alkylthiyl-, Halogen, Biphenyl, Naphtyl-Resten substituierter Phenyl- oder Phenylthiyl-Rest ist.
o = 1 bis 3;
p = 1 bis 4 ist,

wobei die Verbindung der Formel (I') nur eine strahlenhärtende Gruppe aufweist.

**14.** Verwendung der Verbindung der Formel (I) als Schreibmonomer in Photopolymeren, holographischen Medien und/oder holographisch optischen Elementen.

## Claims

**1.** Photopolymer comprising matrix polymers, writing monomers, monomeric fluorourethanes and photoinitiators, wherein the writing monomers comprise compound of formula (I)

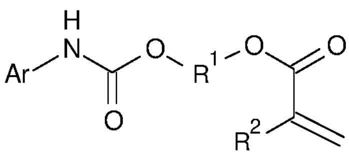

(I);

in which

$R^1$ is an aliphatic hydrocarbyl radical having 1-8 carbon atoms;

$R^2$ is hydrogen or methyl;
Ar is an aromatic radical of the formula (II)

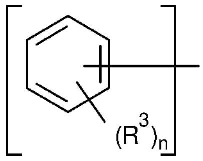

(II)

in which

$R^3$ are independently radicals selected from the group of substituted or unsubstituted phenyl, substituted or unsubstituted phenylthiyl, branched or unbranched alkyl, branched or unbranched alkylthiyl, halogen, where at least one of the $R^3$ radicals is a radical selected from the group of substituted or unsubstituted phenyl, substituted or unsubstituted phenylthiyl;
n = 1 to 5;

or Ar is an aromatic radical of the formula (III)

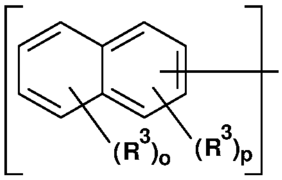

(III)

in which

$R^3$ are independently radicals selected from the group of substituted or unsubstituted phenyl, substituted or unsubstituted phenylthiyl, branched or unbranched alkyl, branched or unbranched alkylthiyl, halogen, where at least one of the $R^3$ radicals is a radical selected from the group of substituted or unsubstituted phenyl, substituted or unsubstituted phenylthiyl;
o = 1 to 3;
p = 1 to 4,

wherein the compound of the formula (I) has only one radiation-curing group.

2. Photopolymer according to Claim 1, **characterized in that** at least one of the $R^3$ radicals is selected from the group of phenyl, phenylthiyl, phenylthiylphenylthiyl, alkylphenyl, alkylphenylthiyl, biphenyl.

3. Photopolymer according to either of Claims 1 and 2, **characterized in that** Ar is a radical of the formula (II).

4. Photopolymer according to any of Claims 1 to 3, **characterized in that** o = 1.

5. Photopolymer according to any of Claims 1 to 4, **characterized in that** $R^1$ is a radical selected from the group of $-CH_2-CH_2-$, $-CH_2-CH_2-CH_2-$, $-CH_2-CHCH_3-$, $-CH_2-CH_2-CH_2-CH_2-$.

6. Photopolymer according to Claims 1-5, **characterized in that** the matrix polymers are crosslinked matrix polymers, preferably three-dimensionally crosslinked matrix polymers and most preferably are three-dimensionally crosslinked polyurethanes.

7. Holographic medium comprising a photopolymer according to any of Claims 1 to 6.

8. Holographic medium according to Claim 7, **characterized in that** it is a film, preferably with a film thickness of 0.3 μm to 500 μm, more preferably with a film thickness of 0.5 μm to 200 μm and yet more preferably with a film

thickness of 1 μm to 100 μm.

**9.** Holographic medium according to either of Claims 7 and 8, **characterized in that** it contains at least one exposed hologram.

**10.** Optical display comprising a holographic medium according to any of Claims 7 to 9.

**11.** Security document comprising a holographic medium according to any of Claims 7 to 9.

**12.** Holographic optical element comprising a holographic medium according to any of Claims 7 to 9.

**13.** Compound of the formula (I')

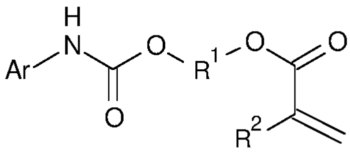

(I');

in which

$R^1$ is an aliphatic hydrocarbyl radical having 1-8 carbon atoms;
$R^2$ is hydrogen or methyl;
Ar is an aromatic radical of the formula (II')

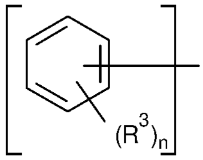

(II')

in which

$R^{3'}$ are independently radicals selected from the group of unsubstituted phenyl, substituted or unsubstituted phenylthiyl, branched or unbranched alkyl, branched or unbranched alkylthiyl, halogen, where at least one of the $R^{3'}$ radicals is a radical selected from the group of unsubstituted phenyl, substituted or unsubstituted phenylthiyl;
n = 1 to 5;

or Ar is an aromatic radical of the formula (III)

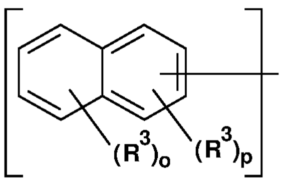

(III)

in which

$R^3$ is a phenyl or phenylthiyl radical substituted by one or more phenyl, phenyl-thiyl, alkyl, alkylthiyl, halogen, biphenyl, naphthyl radicals;
o = 1 to 3;
p = 1 to 4,

wherein the compound of the formula (I') has only one radiation-curing group.

14. Use of the compound of the formula (I) as writing monomer in photopolymers, holographic media and/or holographic optical elements.

## Revendications

1. Photopolymère comprenant des polymères de matrice, des monomères d'écriture, des fluorouréthanes monomériques et des photoinitiateurs, les monomères d'écriture comprenant composé de formule (I)

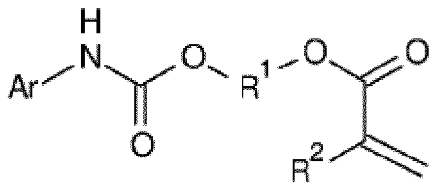

(I);

dans laquelle

$R^1$ est un radical hydrocarboné aliphatique comportant 1 à 8 atomes de C ;
$R^2$ est hydrogène ou méthyle ;
Ar est un radical aromatique de formule (II)

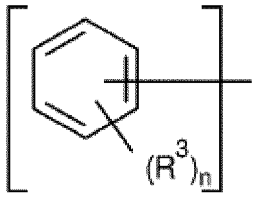

(II),

dans laquelle

$R^3$, indépendamment les uns des autres, sont des radicaux choisis dans le groupe de phényle substitué ou non substitué, phénylthiyle substitué ou non substitué, alkyle ramifié ou non ramifié, alkylthiyle ramifié ou non ramifié, halogène, au moins un des radicaux $R^3$ étant un radical choisi dans le groupe de phényle substitué ou non substitué, phénylthiyle substitué ou non substitué ;
n = 1 à 5 ;

ou Ar est un radical aromatique de formule (III)

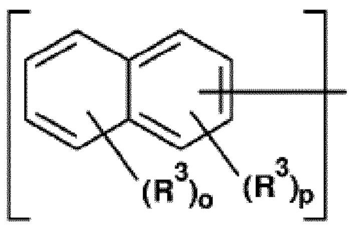

(III),

dans laquelle

$R^3$, indépendamment les uns des autres, sont des radicaux choisis dans le groupe de phényle substitué

ou non substitué, phénylthiyle substitué ou non substitué, alkyle ramifié ou non ramifié, alkylthiyle ramifié ou non ramifié, halogène, au moins un des radicaux $R^3$ étant un radical choisi dans le groupe de phényle substitué ou non substitué, phénylthiyle substitué ou non substitué ;
o = 1 à 3 ;
p = 1 à 4,

le composé de formule (I) ne présentant qu'un seul groupe durcissant par rayonnement.

2. Photopolymère selon la revendication 1, **caractérisé en ce qu'**au moins un des radicaux $R^3$ est choisi dans le groupe de phényle, phénylthiyle, phénylthiylphénylthiyle, alkylphényle, alkylphénylthiyle, biphényle.

3. Photopolymère selon l'une quelconque des revendications 1 et 2, **caractérisé en ce que** Ar est un radical de formule (II).

4. Photopolymère selon l'une quelconque des revendications 1 à 3, **caractérisé en ce que** o = 1.

5. Photopolymère selon l'une quelconque des revendications 1 à 4, **caractérisé en ce que** $R^1$ est un radical choisi dans le groupe $-CH_2-CH_2-$, $-CH_2-CH_2-CH_2-$, $-CH_2-CHCH_3-$, $-CH_2-CH_2-CH_2-CH_2-$.

6. Photopolymère selon les revendications 1 à 5, **caractérisé en ce que** les polymères de matrice sont réticulés, préférablement réticulés tridimensionnellement et tout particulièrement préférablement sont des polyuréthanes réticulés tridimensionnellement.

7. Milieu holographique comprenant un photopolymère selon l'une quelconque des revendications 1 à 6.

8. Milieu holographique selon la revendication 7, **caractérisé en ce qu'**il s'agit d'un film qui peut préférablement présenter une épaisseur de film de 0,3 μm à 500 μm, plus préférablement de 0,5 μm à 200 μm et particulièrement préférablement de 1 μm à 100 μm.

9. Milieu holographique selon l'une quelconque des revendications 7 et 8, **caractérisé en ce qu'**au moins un hologramme est exposé.

10. Affichage optique comprenant un milieu holographique selon l'une quelconque des revendications 7 à 9.

11. Document de sécurité comprenant un milieu holographique selon l'une quelconque des revendications 7 à 9.

12. Élément optique holographique comprenant un milieu holographique selon l'une quelconque des revendications 7 à 9.

13. Composé de formule (I')

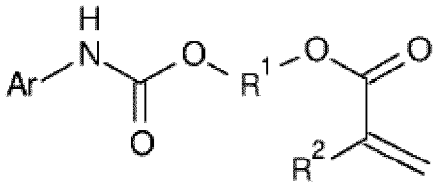

(I');

dans laquelle

$R^1$ est un radical hydrocarboné aliphatique comportant 1 à 8 atomes de C ;
$R^2$ est hydrogène ou méthyle ;
Ar est un radical aromatique de formule (II')

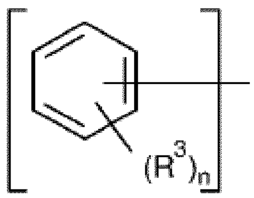

(II'),

dans laquelle

$R^3$, indépendamment les uns des autres, sont des radicaux choisis dans le groupe de phényle non substitué, phénylthiyle substitué ou non substitué, alkyle ramifié ou non ramifié, alkylthiyle ramifié ou non ramifié, halogène, au moins un des radicaux $R^3$ étant un radical choisi dans le groupe de phényle non substitué, phénylthiyle substitué ou non substitué ;
n = 1 à 5 ;

ou Ar est un radical aromatique de formule (III)

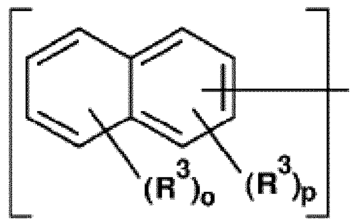

(III),

dans laquelle

$R^3$ est un radical phényle ou phénylthiyle substitué par un ou plusieurs radicaux phényle, phénylthiyle, alkyle, alkylthiyle, halogène, biphényle, naphtyle ;
o = 1 à 3 ;
p = 1 à 4,

le composé de formule (I') ne présentant qu'un seul groupe durcissant par rayonnement.

**14.** Utilisation du composé de formule (I) en tant que monomère d'écriture dans des photopolymères, des milieux holographiques et/ou des éléments optiques holographiques.

Figur 1

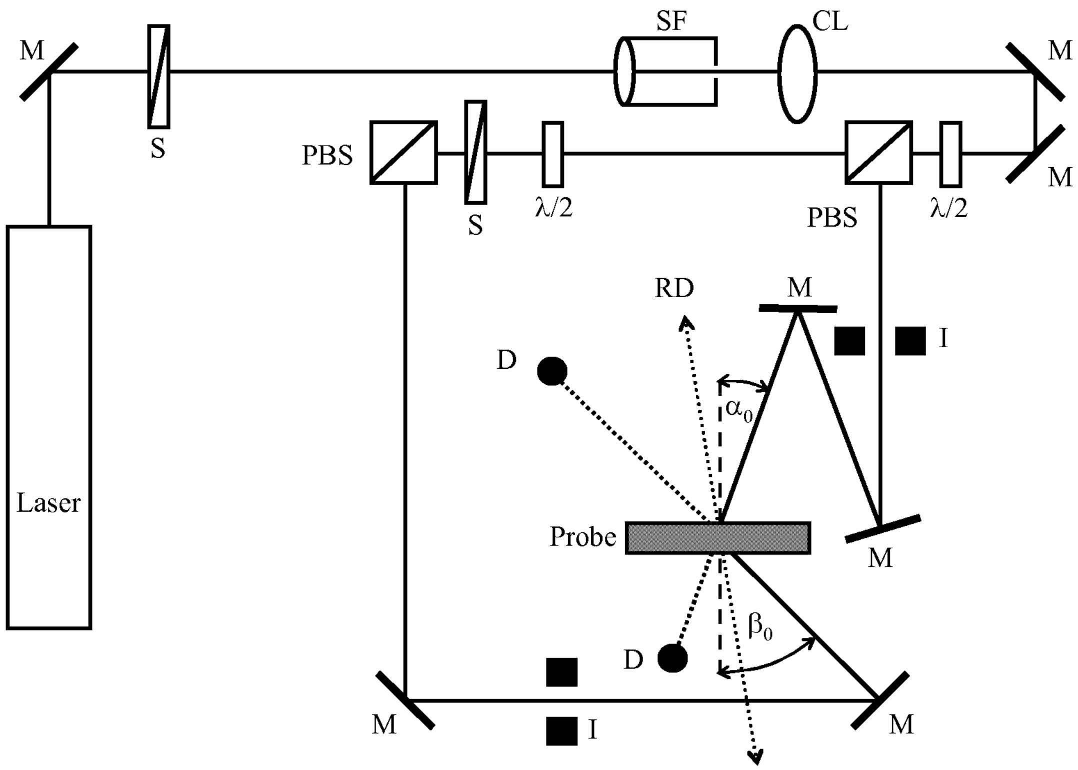

Figur 2

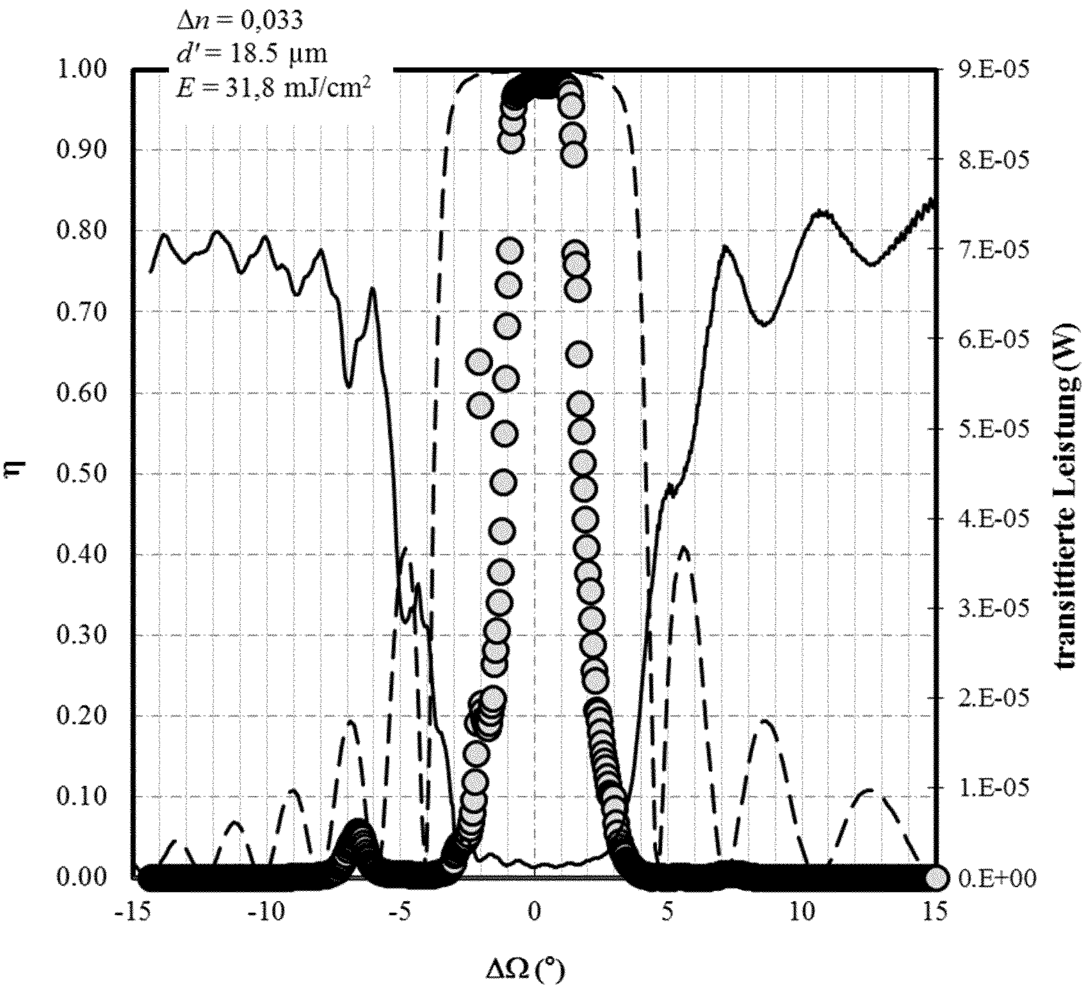

$\Delta n$ = 0,033
$d'$ = 18.5 µm
$E$ = 31,8 mJ/cm²
1.00
0.90
0.80
0.70
0.60
0.50
0.40
0.30
0.20
0.10
0.00
η
9.E-05
8.E-05
7.E-05
6.E-05
5.E-05
4.E-05
3.E-05
2.E-05
1.E-05
0.E+00
transittierte Leistung (W)
-15
-10
-5
0
5
10
15
ΔΩ (°)

Figur 3

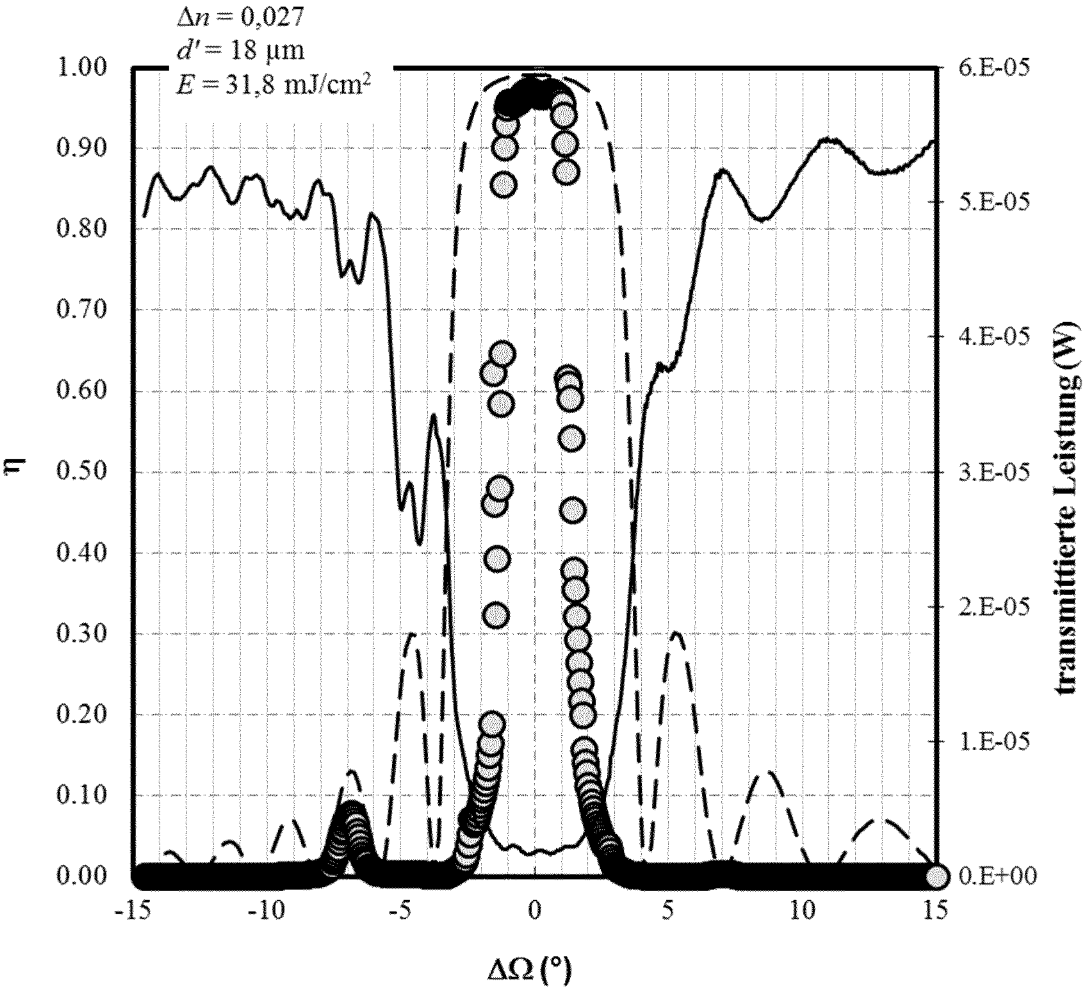

$\Delta n$ = 0,027
$d'$ = 18 µm
$E$ = 31,8 mJ/cm$^2$
1.00
0.90
0.80
0.70
0.60
0.50
0.40
0.30
0.20
0.10
0.00
η
6.E-05
5.E-05
4.E-05
3.E-05
2.E-05
1.E-05
0.E+00
transmittierte Leistung (W)
-15
-10
-5
0
5
10
15
ΔΩ (°)

**Figur 4:**

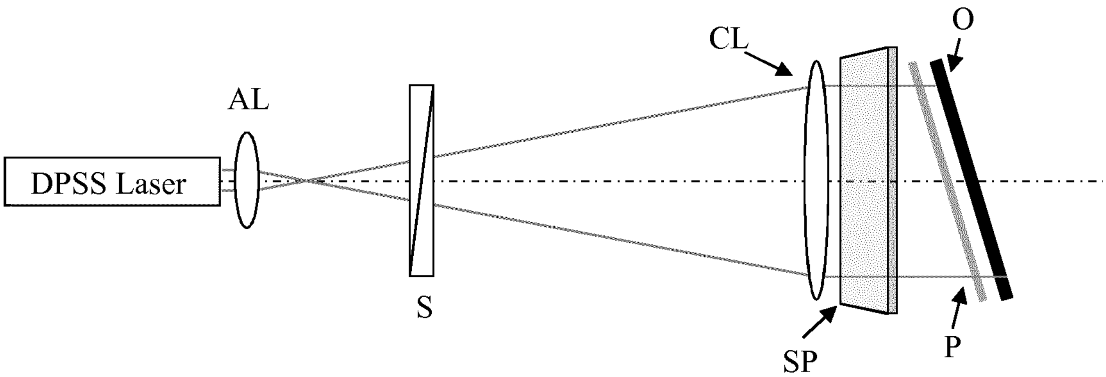

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 20100036013 A
- CN 104371088 A
- EP 2154128 A
- US 4994347 A
- EP 0223587 A
- WO 2012062655 A
- US 20100036013 A1
- US 2012062658 A


### In der Beschreibung aufgeführte Nicht-Patentliteratur


- **P. HARIHARAN.** Optical Holography. Cambridge University Press, 1996
- **HOUBEN-WEYL.** Methoden der organischen Chemie. Georg Thieme Verlag, 1961, vol. XIV/1, 433 ff
- **H. BERNETH ; AZINE DYES.** Ullmann's Encyclopedia of Industrial Chemistry. Wiley-VCH Verlag, 2008
- **H. BERNETH.** Ullmann's Encyclopedia of Industrial Chemistry, Methine Dyes and Pigments. Wiley-VCH Verlag, 2008
- **T. GESSNER ; U. MAYER.** Ullmann's Encyclopedia of Industrial Chemistry, Triarylmethane and Diarylmethane Dyes. Wiley-VCH Verlag, 2000
- *J. Comput. Aid. Mol. Des.,* 2005, vol. 19, 453
- **CUNNINGHAM et al.** *RadTech'98 North America UV/EB Conference Proceedings,* 19. April 1998
- Chemistry & Technology of UV & EB Formulations For Coatings, Inks & Paints. SITA Technology, 1991, vol. 3, 61-328
- **H. KOGELNIK.** *The Bell System Technical Journal,* November 1969, vol. 48 (9), 2909-2947
- *Advanced Synthesis & Catalysis,* 2009, vol. 351 (14+15), 2369-2378